# EUROPEAN PATENT APPLICATION

(11) **EP 0 641 564 A1**
(43) Date of publication of application: **08.03.1995**
(21) Application number: 93910357.8
(22) Date of filing: 19.05.1993
(51) Int. Cl.: A61K 31/415, A61K 31/435, C07D 471/04

(54) **OPTICALLY ACTIVE CONDENSED PYRAZOLE COMPOUND FOR USE IN TREATING THROMBOCYTOPENIA AND ERYTHROPENIA**

(30) Priority: 21.05.1992 JP 154383/92; 02.06.1992 JP 168386/92; 04.02.1993 JP 41990/93; 04.02.1993 JP 41996/93
(71) Applicant: YOSHITOMI PHARMACEUTICAL INDUSTRIES, LTD., Osaka-shi Osaka 541 (JP)
(72) Inventor: HISADOME, Masao Yoshitomi Pharmaceutical Ind Ltd, Yoshitomimachi Chikujo-gun Fukuoka 871 (JP); OE, Takanori, Yoshitomi Pharmaceutical Ind. Ltd., machi, Chikujo-gun Fukuoka 871 (JP); OE, Takanori, Yoshitomi Pharmaceutical Ind. Ltd., Yoshitomimachi,Chikujo-gun Fukuoka 871 (JP)
(74) Representative: von Kreisler, Alek, Dipl.-Chem.
(86) International application number: JP9300656
(87) International publication number: WO9323036

(57) **Abstract**

A remedy for thrombocytopenia or erythropenia containing as the active ingredient a condensed pyrazole compound represented by general formula (I), an optical isomer thereof, or a pharmaceutically acceptable salt thereof, wherein X represents N or CH; R represents hydrogen, alkyl, alkoxy or halogen; Y¹ represents (α) or optionally substituted phenyl; Y² represents (β), -A²-B², hydrogen, alkyl, optionally substituted phenylalkyl, optionally substituted phenyl, alkoxyalkyl, hydroxyalkyl, alkoxycarbonylalkyl, carboxyalkyl or acyloxyalkyl; Z¹ and Z² represent each -O-, -S- or -NR³-, wherein R³ represents hydrogen or alkyl; A¹ and A² represent each alkylene; B¹ and B² represent each carboxy, alkoxycarbonyl, optionally substituted phenylalkoxycarbonyl, acyl, OH, acyloxy, -NR¹R² or -CONR¹R², wherein R¹ and R² represent each hydrogen, alkyl, dialkylaminoalkyl or hydroxyalkyl, or alternatively R¹ and R² represent, together with the adjacent nitrogen atom, a heterocyclic group; and L¹ and L² represent each hydrogen, halogen, alkyl or alkoxy.

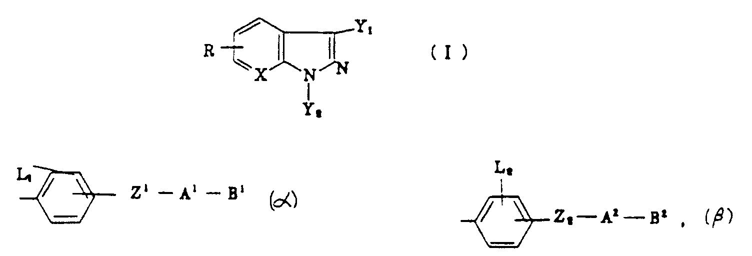

## Description

### Technical Field

The present invention relates to a therapeutic agent for thrombocytopenia and therapeutic agent for erythropenia, which contains a certain fused pyrazole compound, and to a specific, optically active fused pyrazole compound.

### Background Art

Aided by the prominent development of chemotherapeutics, the treatment of infectious diseases has been showing a remarkable progress in recent years. At the same time, however, new problems with respect to opportunistic infectious diseases, on which previous chemotherapeutic agents fail to act effectively, and so on have emerged. Accordingly, a pharmaceutical agent which has a stimulating action on the depression of protection against infection is desired for treating these infectious diseases along with antibacterial drugs.

While drug therapy and radiotherapy are thriving in the treatment of malignant tumor, their side effects are grave to the degree that continued treatment is difficult or patients lose volition to undergo treatments. In particular, myelosuppression (decrease in leukocyte count, platelet count and erythrocyte count) constitutes a major obstacle in the treatment of malignant tumor, and the resolution of such problems has been desired in view of the quality of life of patients.

In the treatment of infectious diseases and malignant tumor, therefore, an integrated treatment, inclusive of a direct treatment of the disease and secondary treatments such as potentiation of preventive action against infection and alleviation of myelosuppression, is needed.

Aplastic anemia, myelodysplastic syndromes, myelogenous anemia, congenital anemia, renal anemia, congenital and idiopathic neutropenia and idiopathic thrombocytopenic purpura are known as incurable diseases caused by myelosuppression. When effective, bone marrow transplantation etc. is done to cure these diseases caused by myelosuppression. However, there have appeared many problems such as limited supply of fresh bone marrow cells and possible induction of viral infection caused by the blood contaminated with various viruses.

At present, human urine-derived colony stimulating factor (M-CSF), granulocyte colony stimulating factor (G-CSF), Romurtide and so on have been developed for use for the treatment of diseases caused by the decrease in leukocyte count, which is a kind of myelosuppression. These drugs exhibit stronger accelerating effect on the recovery of leukocyte count than do conventionally employed glutathione preparations, whereas they may also increase the leukocyte count to an undesired degree and may exhibit no promoting action on the increase of platelet count and erythrocyte count, thus leaving a room for improvement in terms of pharmacological actions. In addition, they pose problems of side effects such as bone ache and fever.

In the absence of an effective drug which directly recovers platelet count for the treatment of the diseases caused by the decrease in platelet count, component transfusion of platelet has been mainly done. Again, the platalet transfusion is subject to the problems of limited supply of fresh platelet and possible induction of viral infection caused by the blood contaminated with various viruses.

For the treatment of diseases caused by the decrease in erythrocyte count, erythropoietin, which is one of the hematopoietic factors, is known as a therapeutic agent. However, erythropoietin is insufficient in that it cannot recover blood counts other than erythrocyte to an effective degree.

Accordingly, there is a strong demand for the development of an efficacious drug having integrated effect on pancytopenia, such as decrease in leukocyte count, platelet count and erythrocyte count, which develops in incurable diseases caused by the aforementioned myelosuppression.

Incidentally, Japanese Patent Unexamined Publication No. 85258/1990 discloses a fused pyrazole compound and a pharmaceutically acceptable salt thereof, having stimulating action on phagocytic activity of leukocyte, stimulating action on phagocytic activity of macrophage, leukocyte count recovery action, stimulating action on resistance to infection, antitumor action, immunological competence-improving action etc.

### Disclosure of the Invention

The present inventors have conducted intensive studies with the aim of solving the above-mentioned problems, and found that the compound disclosed in Japanese Patent Unexamined Publication No. 85258/1990 and a novel optically active compound thereof can accelerate recovery of platelet count and erythrocyte count, which resulted in the completion of the invention.

That is, the present invention relates to an agent for treating thrombocytopenia, containing, as an effective ingredient, a fused pyrazole compound [hereinafter sometimes referred to as Compound (1)] of the formula 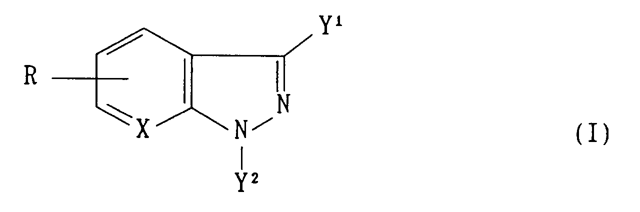
wherein
X is =N- or =CH-;
R is a hydrogen, an alkyl, an alkoxy or a halogen;
Y¹ is a group of the formula 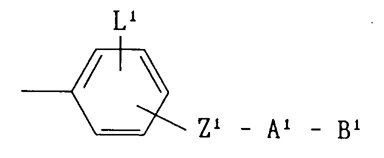
or
a phenyl which may be substituted by 1 to 3 substituent(s) selected from the group of halogen, nitro, amino, alkyl and alkoxy; and
Y² is a group of the formula 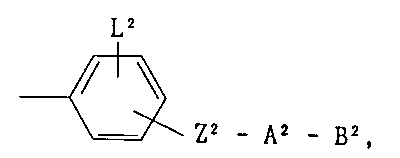
a group of the formula : -A²-B², a hydrogen, an alkyl, a phenylalkyl wherein phenyl nucleus may be substituted by 1 to 3 substituent(s) selected from the group of halogen, nitro, amino, alkyl and alkoxy, a phenyl which may be substituted by 1 to 3 substituent(s) selected from the group of halogen, nitro, amino, alkyl and alkoxy, an alkoxyalkyl, a hydroxyalykl, an alkoxycarbonylalkyl, a carboxyalkyl or an acyloxyalkyl;
provided that either of or both of Y¹ and Y² is(are) represented by the formula 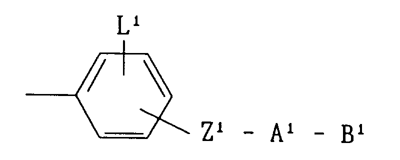
or the formula 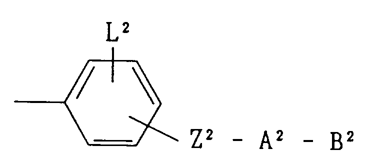
wherein Z¹ and Z² may be the same or different and each is -O-, -S- or -NR³- wherein R³ is a hydrogen or an alkyl, A¹ and A² may be the same or different and each is an alkylene, B¹ and B² may be the same or different and each is a carboxyl, an alkoxycarbonyl, a phenylalkoxycarbonyl wherein phenyl nucleus may be substituted by 1 to 3 substituent(s) selected from the group of halogen, nitro, amino, alkyl and alkoxy, an acyl, a hydroxyl, an acyloxy or a group of the formula 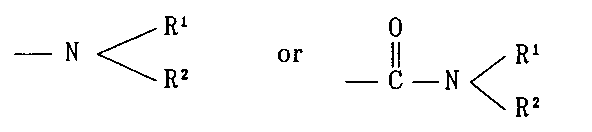
wherein R¹ and R² may be the same or different and each is a hydrogen, an alkyl, a dialkylaminoalkyl or a hydroxyalkyl, or R¹ and R² may form a heterocyclic ring together with the adjoining nitrogen atom, and L¹ and L² may be the same or different and each is a hydrogen, a halogen, an alkyl or an alkoxy, an optical isomer thereof or a pharmaceutically acceptable salt of said compound or the isomer; a method for treating thrombocytopenia, comprising administering Compound (1), an optical isomer thereof or a pharmaceutically acceptable salt of said Compound or the isomer, and the use of Compound (1), an optical isomer thereof or a pharmaceutically acceptable salt of said Compound or the isomer for producing an agent for treating thrombocytopenia.

In addition, the present invention relates to an agent for treating erythropenia, containing, as an active ingredient, Compound (1), an optical isomer thereof or a pharmaceutically acceptable salt of the Compound or the isomer; a method for treating erythropenia comprising administering Compound (1), an optical isomer thereof or a pharmaceutically acceptable salt of the Compound or the isomer, and the use of Compound (1), an optical isomer thereof or a pharmaceutically acceptable salt of the Compound or the isomer for producing an agent for treating erythropenia.

The present invention moreover relates to (S)-3-[4-(2-dimethylamino-1-methylethoxy)phenyl]-1H-pyrazolo[3,4-b]pyridine-1-acetic acid, an ethyl ester thereof and pharmaceutically acceptable salts thereof.

In the present Specification, halogen means chlorine, bromine, fluorine or iodine.

Alkyl means a straight or branched alkyl having 1 to 8 carbon atoms, such as methyl, ethyl, propyl, isopropyl, butyl, isobutyl, tert-butyl, pentyl, isopentyl, hexyl, heptyl and octyl.

Alkoxy means a straight or branched alkoxy having 1 to 8 carbon atoms, such as methoxy, ethoxy, propoxy, isopropoxy, butoxy, isobutoxy, tert-butoxy, pentyloxy, isopentyloxy, hexyloxy, heptyloxy and octyloxy.

Alkylene means a straight or branched alkylene having 1 to 8 carbon atoms, such as methylene, ethylene, 1,1-dimethylethylene, trimethylene, propylene, 2-methyltrimethylene, 2-ethyltrimethylene, tetramethylene, pentamethylene and heptamethylene.

Phenylalkyl means that wherein its alkyl moiety is a straight or branched alkyl having 1 to 4 carbon atoms, which is exemplified by benzyl, 1-phenylethyl, 2-phenylethyl, 3-phenylpropyl and 4-phenylbutyl, and may be substituted, on its phenyl ring, by 1 to 3 substituent(s) selected from the group of halogen, nitro, amino, straight or branched alkyl having 1 to 8 carbon atoms and straight or branched alkoxy having 1 to 8 carbon atoms.

Alkoxyalkyl means that wherein its alkoxy moiety and alkyl moiety are a straight or branched alkoxy having 1 to 4 carbon atoms and a straight or branched alkyl having 1 to 4 carbon atoms, respectively, which is exemplified by methoxymethyl, 2-methoxyethyl, 2-ethoxyethyl, 3-methoxypropyl, 3-ethoxypropyl and 4-ethoxybutyl.

Hydroxyalkyl means that wherein its alkyl moiety is a straight or branched alkyl having 1 to 4 carbon atoms, which is exemplified by 1-hydroxymethyl, 2-hydroxyethyl, 3-hydroxypropyl, 2-hydroxy-1-methylethyl and 2,3-dihydroxypropyl.

Alkoxycarbonylalkyl means that wherein its alkoxy moiety and alkyl moiety are a straight or branched alkoxy having 1 to 4 carbon atoms and a straight or branched alkyl having 1 to 4 carbon atoms, respectively, which is exemplified by methoxycarbonylmethyl, ethoxycarbonylmethyl, 2-methoxycarbonylethyl and 3-ethoxycarbonylpropyl.

Carboxyalkyl means that wherein its alkyl moiety is a straight or branched alkyl having 1 to 4 carbon atoms, which is exemplified by carboxymethyl, 2-carboxyethyl, 1-carboxyethyl and 3-carboxypropyl.

Acyloxyalkyl means that wherein its acyl moiety is a straight or branched alkanoyl having 2 to 5 carbon atoms or benzoyl and its alkyl moiety is a straight or branched alkyl having 1 to 4 carbon atoms, which is exemplified by 2-acetyloxyethyl, 3-acetyloxypropyl and 1-benzoyloxymethyl.

Dialkylaminoalkyl means that wherein each alkyl moiety is a straight or branched alkyl having 1 to 4 carbon atoms, which is exemplified by 2-dimethylaminoethyl, 2-diethylaminoethyl, 3-dimethylaminopropyl and 3-diethylaminopropyl.

Alkoxycarbonyl means that wherein its alkoxy moiety is a straight or branched alkoxy having 1 to 4 carbon atoms, which is exemplified by methoxycarbonyl, ethoxycarbonyl, propoxycarbonyl, isopropoxycarbonyl, butoxycarbonyl and tert-butoxycarbonyl.

Phenylalkoxycarbonyl means that wherein its alkoxy moiety is a straight or branched alkoxy having 1 to 4 carbon atoms, which is exemplified by benzyloxycarbonyl, 2-phenylethoxycarbonyl, 3-phenylpropoxycarbonyl and 4-phenylbutoxycarbonyl and may be substituted, on its phenyl ring, by 1 to 3 substituent(s) selected from the group of halogen, nitro, amino, straight or branched alkyl having 1 to 8 carbon atoms and straight or branched alkoxy having 1 to 8 carbon atoms.

Acyl means straight or branched alkanoyl (e.g. acetyl, propionyl, butyryl, pivaloyl, valeryl) having 2 to 5 carbon atoms or benzoyl.

Acyloxy means that wherein its acyl moiety is a straight or branched alkoxy having 2 to 5 carbon atoms or benzoyl, which is exemplified by acetoxy, propionyloxy, butyryloxy, pivaloyloxy, valeryloxy and benzoyloxy and may be substituted, on its phenyl ring, by 1 to 3 substituent(s) selected from the group of halogen, nitro, amino, straight or branched alkyl having 1 to 8 carbon atoms and straight or branched alkoxy having 1 to 8 carbon atoms.

Heterocyclic ring formed by R¹ and R² together with adjoining nitrogen atom is a 5- to 7-membered ring having, as a hetero atom, at least one nitrogen which may be substituted by alkyl or hydroxyalkyl, or oxygen or sulfur, and is exemplified by 1-pyrrolidinyl, piperidino, 1-piperazinyl, 4-methyl-1-piperazinyl, 4-ethyl-1-piperazinyl, 4-(2-hydroxyethyl)-1-piperazinyl, morpholino, thiomorpholino, 1-homopiperazinyl, 4-methyl-1-homopiperazinyl and 2-oxo-1-pyrrolidinyl.

The pharmaceutically acceptable salts of Compound (1) include, for example, acid addition salts such as hydrochloride, sulfate, hydrobromide, phosphate, formate, acetate, fumarate, maleate, citrate, tartrate, malate, mandelate, methanesulfonate and toluenesulfonate, metal salts such as sodium salt, potassium salt, lithium salt, magnesium salt and calcium salt, amine salts such as ammonium salt and triethylamine salt (inclusive of quaternary ammonium salt) and salts with amino acid such as arginine salt, lysine salt and ornithine salt. Besides these, hydrates such as monohydrate, 1/2 hydrate and 3/2 hydrate and other solvates are also encompassed in the present invention.

The compound of the formula (I) is exemplified by the following compounds.
Ⓞ 1-[4-(3-dimethylaminopropoxy)phenyl]-3-phenyl-1H-pyrazolo[3,4-b]pyridine maleate, melting point 133-136°C
Ⓞ 3-(4-bromophenyl)-1-[4-(2-diethylaminoethoxy)phenyl]-1H-pyrazolo[3,4-b]pyridine maleate, melting point 135-140°C
Ⓞ 1-[3-(3-dimethylamino-2-methylpropoxy)phenyl]-3-phenyl-1H-pyrazolo[3,4-b]pyridine maleate, melting point 134-136°C
Ⓞ 3-[4-(3-dimethylaminopropoxy)phenyl]-1-(4-methylphenyl)-1H-pyrazolo[3,4-b]pyridine maleate, melting point 138-140°C
Ⓞ 3-(4-fluorophenyl)-1-[3-(3-dimethylaminopropoxy)phenyl]-1H-indazole maleate, melting point 110-112°C
Ⓞ N,N-diethyl-3-[3-(4-fluorophenyl)-1H-pyrazolo[3,4-b]pyridin-1-yl]phenoxyacetamide, melting point 102-105°C
Ⓞ 1-[4-(2-dimethylaminoethoxy)phenyl]-3-phenyl-1H-pyrazolo[3,4-b]pyridine maleate, melting point 123-126°C
Ⓞ 1-[4-(4-dimethylaminobutoxy)phenyl]-3-phenyl-1H-pyrazolo[3,4-b]pyridine maleate, melting point 112-115°C
Ⓞ 1-[4-(2-diethylaminoethoxy)phenyl]-3-phenyl-1H-pyrazolo[3,4-b]pyridine maleate, melting point 110-115°C
Ⓞ 1-[4-(3-dimethylamino-2-methylpropoxy)phenyl]-3-phenyl-1H-pyrazolo[3,4-b]pyridine maleate, melting point 143-146°C
Ⓞ 3-(4-chlorophenyl)-1-[4-(3-dimethylaminopropoxy)phenyl]-1H-pyrazolo[3,4-b]pyridine maleate, melting point 162-165°C
Ⓞ 3-(4-bromophenyl)-1-[4-(3-dimethylaminopropoxy)phenyl]-1H-pyrazolo[3,4-b]pyridine maleate, melting point 162-165°C
Ⓞ 1-[3-(2-dimethylaminoethoxy)phenyl]-3-phenyl-1H-pyrazolo[3,4-b]pyridine dimaleate, melting point 117-120°C
Ⓞ 1-[3-(3-dimethylaminopropoxy)phenyl]-3-phenyl-1H-pyrazolo[3,4-b]pyridine maleate, melting point 120-123°C
Ⓞ 1-[3-(2-morpholinoethoxy)phenyl]-3-phenyl-1H-pyrazolo[3,4-b]pyridine maleate, melting point 140-142°C
Ⓞ 3-(4-chlorophenyl)-1-[3-(2-dimethylaminoethoxy)phenyl]-1H-pyrazolo[3,4-b]pyridine maleate, melting point 140-143°C
Ⓞ 1-[2-(2-dimethylaminoethoxy)phenyl]-3-phenyl-1H-pyrazolo[3,4-b]pyridine maleate, melting point 153-157°C
Ⓞ 1-[2-(3-dimethylaminopropoxy)phenyl]-3-phenyl-1H-pyrazolo[3,4-b]pyridine maleate, melting point 123-125°C
Ⓞ 3-(4-fluorophenyl)-1-[3-(2-piperidinoethoxy)phenyl]-1H-pyrazolo[3,4-b]pyridine maleate, melting point 149-151°C
Ⓞ 3-(4-fluorophenyl)-1-[3-(3-dimethylaminopropoxy)phenyl]-1H-pyrazolo[3,4-b]pyridine maleate 1/2 hydrate, melting point 102-105°C
Ⓞ 3-(4-chlorophenyl)-1-[3-(3-dimethylamino-2-methylpropoxy)phenyl]-1H-pyrazolo[3,4-b]pyridine maleate, melting point 150-152°C
Ⓞ 1-[3-(2-diethylaminoethoxy)phenyl]-3-phenyl-1H-pyrazolo[3,4-b]pyridine maleate 1/2 hydrate, melting point 86-90°C
Ⓞ 3-(4-fluorophenyl)-1-[3-(3-piperidinopropoxy)phenyl]-1H-pyrazolo[3,4-b]pyridine fumarate, melting point 73-75°C
Ⓞ 3-(4-fluorophenyl)-1-[3-(3-dimethylamino-2-methylpropoxy)phenyl]-1H-pyrazolo[3,4-b]pyridine maleate 1/2 hydrate, melting point 128-130°C
Ⓞ 3-(4-fluorophenyl)-1-[3-(3-dimethylaminopropoxy)phenyl]-1H-pyrazolo[3,4-b]pyridine maleate, melting point 110-112°C
Ⓞ 3-(4-chlorophenyl)-1-[3-(3-dimethylaminopropoxy)phenyl]-1H-pyrazolo[3,4-b]pyridine fumarate, melting point 170-172°C
Ⓞ 1-[3⁻(4-dimethylaminobutoxy)phenyl]-3-phenyl-1H-pyrazolo[3,4-b]pyridine fumarate hydrate, melting point 140-143°C
Ⓞ 3-[4-(3-dimethylaminopropoxy)phenyl]-1-phenyl-1H-pyrazolo[3,4-b]pyridine maleate, melting point 132-135°C
Ⓞ 3-[4-(2-dimethylaminoethoxy)phenyl]-1-phenyl-1H-pyrazolo[3,4-b]pyridine maleate, melting point 137-141°C
Ⓞ 4-{3-[3-(4-fluorophenyl)-1H-pyrazolo[3,4-b]pyridin-1-yl]phenoxyacetyl}morpholine, melting point 141-143°C
Ⓞ 1-(4-methylphenyl)-3-[4-(3-dimethylamino-2-methylpropoxy)phenyl]-1H-pyrazolo[3,4-b]pyridine maleate, melting point 170-171°C
Ⓞ 1-methyl-3-[4-(3-dimethylaminopropoxy)phenyl]-1H-pyrazolo[3,4-b]pyridine maleate, melting point 128-129°C
Ⓞ 6-methyl-1-[3-(3-dimethylamino-2-methylpropoxy)phenyl]-3-phenyl-1H-pyrazolo[3,4-b]pyridine maleate, melting point 123-125°C
Ⓞ 1-[3-(3-dimethylamino-2-methylpropoxy)phenyl]-3-phenyl-1H-indazole maleate 2/5 ethyl acetate, melting point 58-63°C
Ⓞ 1-[4-(3-hydroxypropoxy)phenyl]-3-phenyl-1H-pyrazolo[3,4-b]pyridine, melting point 135-138°C
Ⓞ N-(2-dimethylaminoethyl)-4-(1-phenyl-1H-pyrazolo[3,4-b]pyridin-3-yl)phenoxyacetamide maleate, melting point 169-171°C
Ⓞ N-(2-hydroxyethyl)-3-[3-(4-fluorophenyl)-1H-pyrazolo[3,4-b]pyridin-1-yl]phenoxyacetamide, melting point 161-163°C
Ⓞ N-(2-diethylaminoethyl)-3-[3-(4-fluorophenyl)-1H-pyrazolo[3,4-b]pyridin-1-yl]phenoxyacetamide, melting point 137-138°C
Ⓞ 1-{3-[3-(4-fluorophenyl)-1H-pyrazolo[3,4-b]pyridin-1-yl]phenoxyacetyl}-4-methylpiperazine, melting point 132-135°C
Ⓞ N-(2-diethylaminoethyl)-4-(1-phenyl-1H-pyrazolo[3,4-b]pyridin-3-yl)phenoxyacetamide fumarate, melting point 152-154°C
Ⓞ 4-methyl-1-[4-(1-phenyl-1H-pyrazolo[3,4-b]pyridin-3-yl)phenoxyacetyl]piperazine maleate, melting point 187-189°C
Ⓞ 3-[4-(3-dimethylaminopropoxy)phenyl]-1H-pyrazolo[3,4-b]pyridine, melting point 176-177°C
Ⓞ 3-[4-(2-dimethylaminoethylamino)phenyl]-1H-pyrazolo[3,4-b]pyridine, melting point 165-167°C
Ⓞ 1-methyl-3-[4-(2-dimethylaminoethylamino)phenyl]-1H-pyrazolo[3,4-b]pyridine maleate, melting point 88-90°C
Ⓞ 3-[4-(2-dimethylaminoethoxy)phenyl]-1H-pyrazolo[3,4-b]pyridine, melting point 132-134°C
Ⓞ 1-(4-chlorobenzyl)-3-[4-(3-dimethylaminopropoxy)phenyl]-1H-pyrazolo[3,4-b]pyridine maleate, melting point 136-138°C
Ⓞ 3-[4-(2-dimethylaminoethoxy)phenyl]-1-(2-methoxyethyl)-1H-pyrazolo[3,4-b]pyridine maleate, melting point 115-117°C
Ⓞ N,N-dimethyl-3-[4-(3-dimethylaminopropoxy)phenyl]-1H-pyrazolo[3,4-b]pyridin-1-ylacetamide maleate, melting point 165-167°C
Ⓞ 1-(2,4-dichlorobenzyl)-3-[4-(2-dimethylaminoethoxy)phenyl]-1H-pyrazolo[3,4-b]pyridine maleate, melting point 136-138°C
Ⓞ 1-butyl-3-[4-(3-dimethylaminopropoxy)phenyl]-1H-pyrazolo[3,4-b]pyridine maleate, melting point 112-113°C
Ⓞ ethyl 3-[4-(3-dimethylaminopropoxy)phenyl]-1H-pyrazolo[3,4-b]pyridine-1-acetate maleate, melting point 108-110°C
Ⓞ 1-acetonitrile-3-[4-(3-dimethylaminopropoxy)phenyl]-1H-pyrazolo[3,4-b]pyridine maleate, melting point 119-121°C
Ⓞ 1-(4-fluorophenacyl)-3-[4-(3-dimethylaminopropoxy)phenyl]-1H-pyrazolo[3,4-b]pyridine 3/2 fumarate, melting point 165-169°C
Ⓞ 1-(2-chlorobenzyl)-3-[4-(2-dimethylaminoethylamino)phenyl]-1H-pyrazolo[3,4-b]pyridine fumarate, melting point 155-157°C
Ⓞ 1-(2-dimethylaminoethyl)-3-[4-(2-dimethylaminoethoxy)phenyl]-1H-pyrazolo[3,4-b]pyridine dimaleate, melting point 96-98°C
Ⓞ 1-[3-(3-dimethylaminopropoxy)phenyl]-3-[4-(3-dimethylaminopropoxy)phenyl]-1H-pyrazolo[3,4-b]pyridine difumarate, melting point 145-148°C
Ⓞ 1-(3-dimethylaminopropyl)-3-[4-(3-dimethylaminopropoxy)phenyl]-1H-pyrazolo[3,4-b]pyridine dimaleate, melting point 127-130°C
Ⓞ 1-[3-(2-dimethylaminoethoxy)phenyl]-3-[4-(2-dimethylaminoethoxy)phenyl]-1H-pyrazolo[3,4-b]pyridine difumarate, melting point 115-118°C
Ⓞ 1-(4-methylphenyl)-3-[4-(2-dimethylamino-1-methylethoxy)phenyl]-1H-pyrazolo[3,4-b]pyridine maleate, melting point 139-140°C
Ⓞ 6-methyl-1-(4-methylphenyl)-3-[4-(3-dimethylaminopropoxy)phenyl]-1H-pyrazolo[3,4-b]pyridine
Ⓞ 6-methyl-1-(4-methylphenyl)-3-[4-(3-dimethylamino-2-methylpropoxy)phenyl]-1H-pyrazolo[3,4-b]pyridine
Ⓞ 1-(3-methylphenyl)-3-[4-(3-dimethylaminopropoxy)phenyl]-1H-pyrazolo[3,4-b]pyridine
Ⓞ 1-(3-methylphenyl)-3-[4-(3-dimethylamino-2-methylpropoxy)phenyl]-1H-pyrazolo[3,4-b]pyridine
Ⓞ 1-(2-methylphenyl)-3-[4-(3-dimethylaminopropoxy)phenyl]-1H-pyrazolo[3,4-b]pyridine
Ⓞ 1-(2-methylphenyl)-3-[4-(3-dimethylamino-2-methylpropoxy)phenyl]-1H-pyrazolo[3,4-b]pyridine
Ⓞ 1-[3-(2-dimethylamino-1-methylethoxy)phenyl]-3-phenyl-1H-pyrazolo[3,4-b]pyridine maleate, melting point 118-120°C
Ⓞ 2-(4-methylphenyl)-3-[4-(2-dimethylamino-2-methylethoxy)phenyl]-1H-pyrazolo[3,4-b]pyridine maleate, melting point 126-128°C
Ⓞ 1-[3-(2-dimethylamino-2-methylethoxy)phenyl]-3-phenyl-1H-pyrazolo[3,4-b]pyridine 3/2 fumarate, melting point 139-142°C
Ⓞ 3-(4-fluorophenyl)-1-[3-(2-dimethylamino-1-methylethoxy)phenyl]-1H-pyrazolo[3,4-b]pyridine fumarate, melting point 148-149°C
Ⓞ 3-(4-fluorophenyl)-1-[3-(2-dimethylamino-2-methylethoxy)phenyl]-1H-pyrazolo[3,4-b]pyridine 3/2 fumarate, melting point 174-176°C
Ⓞ 3-(4-chlorophenyl)-1-[3-(2-dimethylamino-1-methylethoxy)phenyl]-1H-pyrazolo[3,4-b]pyridine fumarate, melting point 150-152°C
Ⓞ 3-(4-chlorophenyl)-1-[3-(2-dimethylamino-2-methylethoxy)phenyl]-1H-pyrazolo[3,4-b]pyridine 3/2 fumarate, melting point 148-150°C
Ⓞ 1-[3-(3-dimethylamino-2-methylpropoxy)phenyl]-1-phenyl-1H-pyrazolo[3,4-b]pyridine maleate 2/5 ethyl acetate, melting point 58-60°C
Ⓞ 1-(2-hydroxyethyl)-3-[4-(3-dimethylaminopropoxy)phenyl]-1H-pyrazolo[3,4-b]pyridine 1/2 fumarate, melting point 162-163°C
Ⓞ 1-(2-acetoxyethyl)-3-[4-(3-dimethylaminopropoxy)phenyl]-1H-pyrazolo[3,4-b]pyridine fumarate, melting point 122-124°C
Ⓞ N-methyl-3-[4-(3-dimethylaminopropoxy)phenyl]-1H-pyrazolo[3,4-b]pyridin-1-ylacetamide difumarate, melting point 102-105°C
Ⓞ methyl 6-{3-[4-(3-dimethylaminopropoxy)phenyl]-1H-pyrazolo[3,4-b]pyridin-1-yl}hexanate,
NMR(CDCl₃) δ: 1.33-2.70 (m,12H), 2.24 (s,6H), 3.60 (s,3H), 4.07 (t,2H), 4.53 (t,2H), 6.93-8.51 (7H)
Ⓞ ethyl 3-[4-(2-dimethylaminoethoxy)phenyl]-1H-pyrazolo[3,4-b]pyridine-1-acetate maleate, melting point 138-140°C
Ⓞ ethyl 4-{3-[4-(3-dimethylaminopropoxy)phenyl]-1H-pyrazolo[3,4-b]pyridin-1-yl}butyrate difumarate, melting point 121-124°C
Ⓞ ethyl 3-{3-[4-(3-dimethylaminopropoxy)phenyl]-1H-pyrazolo[3,4-b]pyridin-1-y}propionate fumarate, melting point 105-107°C
Ⓞ ethyl 2-methyl-2-{3-[4-(3-dimethylaminopropoxy)phenyl]-1H-pyrazolo[3,4-b]pyridin-1-yl}propionate,
NMR(CDCl₃) δ: 1.10 (t,3H), 2.03 (s,6H), 1.96-2.40 (m,2H), 2.30 (s,6H), 2.52 (t,2H), 4.01 (t,2H), 4.12 (q,2H), 6.98-8.51 (7H)
Ⓞ ethyl 3-[4-(2-dimethylamino-1-methylethoxy)phenyl]-1H-pyrazolo[3,4-b]pyridine-1-acetate,
NMR(CDCl₃) δ: 1.25 (t,3H), 1.33 (d,3H), 2.33 (s,6H), 2.34-2.78 (m,2H), 4.21 (q,2H), 4.55 (q,1H), 5.26 (s,2H), 6.98-8.51 (7H)
Ⓞ ethyl 4-(1-phenyl-1H-pyrazolo[3,4-b]pyridin-3-yl)phenoxyacetate, melting point 118-120°C
Ⓞ 4-(1-phenyl-1H-pyrazolo[3,4-b]pyridin-3-yl)phenoxyacetic acid, melting point 220-222°C
Ⓞ ethyl 3-[3-(4-fluorophenyl)-1H-pyrazolo[3,4-b]pyridin-1-yl]phenoxyacetate, melting point 130-131°C
Ⓞ 3-[3-(4-fluorophenyl)-1H-pyrazolo[3,4-b]pyridin-1-yl]phenoxyacetic acid, melting point 225-226°C
Ⓞ ethyl 4-(3-phenyl-1H-pyrazolo[3,4-b]pyridin-1-yl)phenoxyacetate
Ⓞ 4-(3-phenyl-1H-pyrazolo[3,4-b]pyridin-1-yl)phenoxyacetic acid
Ⓞ 3-[4-(3-dimethylaminopropoxy)phenyl]-1-propionyl-1H-pyrazolo[3,4-b]pyridine 3/2 fumarate, melting point 155-157°C
Ⓞ 3-[4-(3-dimethylaminopropoxy)phenyl]-1H-pyrazolo[3,4-b]pyridine-1-acetic acid, melting point 221-223°C
Ⓞ 3-[4-(2-dimethylamino-1-methylethoxy)phenyl]-1H-pyrazolo[3,4-b]pyridine, melting point 130-132°C
Ⓞ ethyl (S)-3-[4-(2-dimethylamino-1-methylethoxy)phenyl]-1H-pyrazolo[3,4-b]pyridine-1-acetate, melting point 165-166°C
Ⓞ 3-[4-(2-dimethylamino-1-methylethoxy)phenyl]-1H-pyrazolo[3,4-b]pyridine-1-acetic acid, melting point 210-211°C
Ⓞ (S)-3-[4-(2-dimethylamino-1-methylethoxy)phenyl]-1H-pyrazolo[3,4-b]pyridine-1-acetic acid, melting point 212-213°C
Ⓞ 3-[4-(3-dimethylaminopropoxy)phenyl]-6-isopropyl-1H-pyrazolo[3,4-b]pyridine-1-acetic acid
Ⓞ ethyl 3-[4-(3-dimethylaminopropoxy)phenyl]-6-isopropyl-1H-pyrazolo[3,4-b]pyridine-1-acetate
Ⓞ 3-[4-(2-dimethylamino-1-methylethoxy)phenyl]-6-isopropyl-1H-pyrazolo[3,4-b]pyridine-1-acetic acid
Ⓞ (S)-3-[4-(2-dimethylamino-1-methylethoxy)phenyl]-6-isopropyl-1H-pyrazolo[3,4-b]pyridine-1-acetic acid
Ⓞ ethyl 3-[4-(2-dimethylamino-1-methylethoxy)phenyl]-6-isopropyl-1H-pyrazolo[3,4-b]pyridine-1-acetate
Ⓞ ethyl (S)-3-[4-(2-dimethylamino-1-methylethoxy)phenyl]-6-isopropyl-1H-pyrazolo[3,4-b]pyridine-1-acetate
Ⓞ 6-methyl-3-[4-(3-dimethylaminopropoxy)phenyl]-1H-pyrazolo[3,4-b]pyridine-1-acetic acid
Ⓞ ethyl 6-methyl-3-[4-(3-dimethylaminopropoxy)phenyl]-1H-pyrazolo[3,4-b]pyridine-1-acetate
Ⓞ 2-methyl-2-{3-[4-(3-dimethylaminopropoxy)phenyl]-1H-pyrazolo[3,4-b]pyridin-1-yl}propionic acid, melting point 187-190°C
Ⓞ 3-{3-[4-(3-dimethylaminopropoxy)phenyl]-1H-pyrazolo[3,4-b]pyridin-1-yl}propionic acid 1/2 hydrate, melting point 86-90°C
Ⓞ 6-{3-[4-(3-dimethylaminopropoxy)phenyl]-1H-pyrazolo[3,4-b]pyridin-1-yl}hexanoic acid, melting point 96-100°C
Ⓞ 3-[4-(2-dimethylaminoethylamino)phenyl]-1H-pyrazolo[3,4-b]pyridine-1-acetic acid,
NMR(CDCl₃) δ: 1.20 (t,3H), 2.40 (s,6H), 2.56 (t,2H), 3.20 (t,2H), 4.20 (q,2H), 5.30 (s,2H), 6.63-8.56 (7H)
Ⓞ 3-[4-(2-dimethylaminoethylamino)phenyl]-1H-pyrazolo[3,4-b]pyridine-1-acetic acid, melting point 240-242°C
Ⓞ 3-phenyl-1-(4-trifluoroacetylaminophenyl)-1H-pyrazolo[3,4-b]pyridine, melting point 172-174°C
Ⓞ 1-[4-(2-dimethylaminoethylamino)phenyl]-3-phenyl-1H-pyrazolo[3,4-b]pyridine maleate 1/2 hydrate, melting point 80-88°C
Ⓞ 1-[4-(3-dimethylaminopropylamino)phenyl]-3-phenyl-1H-pyrazolo[3,4-b]pyridine dimaleate, melting point 145-146°C
A compound of the formula (I) wherein X is =N- and/or at least one of Z¹ and Z² is oxygen is preferable.

Preferable examples of the compound of the formula (I) and a pharmaceutically acceptable salt thereof are given in the following.
Ⓞ 3-[4-(2-dimethylamino-1-methylethoxy)phenyl]-1H-pyrazolo[3,4-b]pyridine-1-acetic acid,
Ⓞ ethyl 3-[4-(2-dimethylamino-1-methylethoxy)phenyl]-1H-pyrazolo[3,4-b]pyridine-1-acetate,
Ⓞ 3-[4-(3-dimethylaminopropoxy)phenyl]-6-isopropyl-1H-pyrazolo[3,4-b]pyridine-1-acetic acid,
Ⓞ ethyl 3-[4-(3-dimethylaminopropoxy)phenyl]-6-isopropyl-1H-pyrazolo[3,4-b]pyridine-1-acetate,
Ⓞ 3-[4-(2-dimethylamino-1-methylethoxy)phenyl]-6-isopropyl-1H-pyrazolo[3,4-b]pyridine-1-acetic acid,
Ⓞ ethyl 3-[4-(2-dimethylamino-1-methylethoxy)phenyl]-6-isopropyl-1H-pyrazolo[3,4-b]pyridine-1-acetate,
Ⓞ 6-methyl-3-[4-(3-dimethylaminopropoxy)phenyl]-1H-pyrazolo[3,4-b]pyridine-1-acetic acid,
Ⓞ ethyl 6-methyl-3-[4-(3-dimethylaminopropoxy)phenyl]-1H-pyrazolo[3,4-b]pyridine-1-acetate,
optical isomers thereof and pharmaceutically acceptable salts thereof.

Most of the Compounds (1) to be used in the present invention are produced by the method described in Japanese Patent Unexamined Publication No. 85258/1990 and this gazette is hereby cited for reference. The optical isomers thereof can be obtained by using optically active materials or optical resolution of the obtained racemates.

The compounds to be used as the active ingredient for achieving the object of the present invention are particularly preferably 3-[4-(2-dimethylamino-1-methylethoxy)phenyl]-1H-pyrazolo[3,4-b]pyridine-1-acetic acid, an ethyl ester thereof, optically active compounds thereof and pharmaceutically acceptable salts thereof.

The compounds useful for achieving the object of the present invention are novel (S)-3-[4-(2-dimethylamino-1-methylethoxy)phenyl]-1H-pyrazolo[3,4-b]pyridine-1-acetic acid, an ethyl ester thereof and pharmaceutically acceptable salts thereof. Optically active compounds thereof can be produced by the following method.

That is, an optically active (S)-1-dimethylamino-2-propanol known from Japanese Patent Publication No. 19252/1974 and, for example, 2-chloro-3-(4-fluorobenzoyl)pyridine are reacted in a solvent inert to the reaction, such as benzene, toluene, xylene, pyridine, ethanol, isopropyl alcohol, ethylene glycol dimethyl ether, dimethylformamide or dioxane with the use of, where necessary, a deacidifying agent such as sodium hydroxide, sodium hydride, potassium hydride, sodium hydrogencarbonate, sodium carbonate, potassium carbonate or triethylamine at a temperature between 0°C and 250°C to give (S)-2-chloro-3-[4-(2-dimethylamino-1-methylethoxy)benzoyl]pyridine and said compound and a hydrazine hydrate are refluxed in a suitable solvent such as benzene, toluene, xylene, dioxane, diethylene glycol dimethyl ether or pyridine with the use of, where necessary, a suitable dehydrating agent or a deacidifying agent, whereby (S)-3-[4-(2-dimethylamino-1-methylethoxy)phenyl]-1H-pyrazolo[3,4-b]pyridine is obtained.

The thus-obtained pyrazolo[3,4-b]pyridine compound and ethyl chloroacetate are reacted in a solvent inert to the reaction, such as benzene, toluene, xylene, dimethylformamide, pyridine, chloroform, dichloromethane, dichloroethane, methanol or ethanol with the use of, where necessary, a deacidifying agent such as sodium hydroxide, potassium hydroxide, sodium hydride, potassium hydride, sodium carbonate, potassium carbonate or triethylamine at a temperature between 0°C and 250°C, whereby ethyl (S)-3-[4-(2-dimethylamino-1-methylethoxy)phenyl]-1H-pyrazolo[3,4-b]pyridine-1-acetate is obtained.

Further, said ethyl acetate compound is hydrolyzed in the presence of an acid or an alkali to give (S)-3-[4-(2-dimethylamino-1-methylethoxy)phenyl]-1H-pyrazolo[3,4-b]pyridine-1-acetic acid.

The Compounds (1) and optically active compounds thereof inclusive of the thus-obtained 3-[4-(2-dimethylamino-1-methylethoxy)phenyl]-1H-pyrazolo[3,4-b]pyridine-1-acetic acid and an ethyl ester compound thereof can be converted to the corresponding acid addition salts by a treatment with inorganic acid, organic acid etc. such as hydrochloric acid, sulfuric acid, hydrobromic acid, phosphoric acid, formic acid, acetic acid, fumaric acid, maleic acid, citric acid, tartaric acid, malic acid, mandelic acid, methanesulfonic acid and toluenesulfonic acid by conventional methods.

Moreover, (S)-3-[4-(2-dimethylamino-1-methylethoxy)phenyl]-1H-pyrazolo[3,4-b]pyridine-1-acetic acid ethyl ester can be formulated into an oral preparation, since it shows superior absorption by oral administration and superior bioavailability.

The phamacological action of the compound of the present invention will be clarified by the following experiments.

Compound A, Compound B and Compound C used in Experimental Examples are the following compounds.
- Compound A: = 3-[4-(2-dimethylamino-1-methylethoxy)phenyl]-1H-pyrazolo[3,4-b]pyridine-1-acetic acid
- Compound B: = (S)-3-[4-(2-dimethylamino-1-methylethoxy)phenyl]-1H-pyrazolo[3,4-b]pyridine-1-acetic acid
- Compound C: = ethyl (S)-3-[4-(2-dimethylamino-1-methylethoxy)phenyl]-1H-pyrazolo[3,4-b]pyridine-1-acetate hydrochloride

### Experimental Example 1 : Promoting action on recovery of platelet count in mice transplanted with bone marrow

ICR mice (6-8 weeks old) were exposed to 8.0 Gy total body irradiation and 10⁷ bone marrow cells from the mice of the same species were transplanted to the mice. Then, Compound A or Compound B was intravenously administered for 9 consecutive days starting from the next day of the bone marrow transplantation. On day 11 after the bone marrow transplantation, heparinized blood was taken from the abdominal aorta of mice and platelet count was measured using an automatic cell counter. The experiment was done with 5 mice per group. The difference in platelet count between an untreated group and the bone marrow transplanted group was taken as 100%, based on which increase in the platelet count was calculated.

**Table 1**

| Pretreatment | Test compound | Dose (mg/kg) | Platelet count (10⁴/mm³) | Increase in platelet count (%) |
|---|---|---|---|---|
| X-irradiation alone | - | 0 | 6.7±2.9 | - |
| X-irradiation+bone marrow transplantation | - | 0 | 16.2±4.4 | 0 |
| X-irradiation+bone marrow transplantation | Comp. A | 0.01 | 27.4±9.6 | 30 |
| X-irradiation+bone marrow transplantation | Comp. A | 0.03 | 31.4±11.4 | 41 |
| X-irradiation+bone marrow transplantation | Comp. A | 0.1 | 42.3±10.6 | 71 |
| X-irradiation+bone marrow transplantation | Comp. A | 0.3 | 49.8±9.7 | 91 |
| no treatment | - | 0 | 53.0±17.9 | 100 |

As is evident from Table 1, administration of Compound A to bone marrow transplanted mice resulted in the recovery of the platelet count to an almost normal level.

**Table 2**

| Pretreatment | Test compound | Dose (mg/kg) | Platelet count (10⁴/mm³) | Increase in platelet count (%) |
|---|---|---|---|---|
| X-irradiation alone | - | 0 | 5.1±1.2 | - |
| X-irradiation+bone marrow transplantation | - | 0 | 9.4±3.4 | 0 |
| X-irradiation+bone marrow transplantation | Comp. B | 0.01 | 45.9±6.7 | 65 |
| X-irradiation+bone marrow transplantation | Comp. B | 0.03 | 54.8±13.6 | 81 |
| no treatment | - | 0 | 65.2±13.2 | 100 |

As is evident from Table 2, administration of Compound B to bone marrow transplanted mice resulted in the recovery of the platelet count to an almost normal level.

### Experimental Example 2 : Promoting action on recovery of decreased platelet count in rats administered with anti-tumor drug

An anti-tumor drug, nimustine hydrochloride, (30 mg/kg) was intravenously administered to SD rats (6 weeks old) and 15 mg/kg thereof was intravenously administered the next day. Compound A was intravenously administered for 4 days on day 8, day 9, day 10 and day 14 after the initial administration of nimustine hydrochloride. On certain days after the administration, heparinized blood was taken from the rat tail vein and platelet count was measured using an automatic cell counter. The experiment was done with 4-6 rats per group.

**Table 3**

| Pretreatment | Test compound | Dose (mg/kg) | Platelet count (10⁴/mm³) | |
|---|---|---|---|---|
| | | | day 25 | day 44 |
| nimustine hydrochloride alone | - | 0 | 8.8±3.8 | 22.7±12.8 |
| nimustine hydrochloride | Comp. A | 1.0 | 17.7±21.8 | 57.3±20.2 |

As is evident from Table 3, administration of Compound A to the mice administered with nimustine hydrochloride resulted in promoted recovery of the decreased platelet count.

Based on the foregoing Experimental Examples, it is evident that administration of Compound A to animals transplanted with bone marrow or administered with nimustine hydrochloride results in promoted recovery of decreased platelet count.

### Experimental Example 3 : Promoting action on recovery of erythrocyte count in mice irradiated with X-rays (preventive effects)

BALB/c mice (4-5 weeks old) were exposed to 4.0 Gy total body irradiation. Compound A was intravenously administered for 3 consecutive days starting from 3 days before and up to the previous day of the irradiation. On day 19 and day 22 after the X-ray irradiation, blood was taken from orbital vein plexus of mice and erythrocyte count was measured using an automatic cell counter. The experiment was done with 8 mice per group and the results are shown as mean value±standard deviation.

**Table 4**

| Treatment | Test compound | Dose (mg/kg) | Erythrocyte count (10⁴/mm³) | |
|---|---|---|---|---|
| | | | day 19 | day 22 |
| X-irradiation | - | 0 | 506±175 | 696±192 |
| X-irradiation | Comp. A | 0.01 | 835±102 | 904±45 |
| X-irradiation | Comp. A | 0.1 | 837±118 | 916±86 |
| X-irradiation | Comp. A | 1 | 856±118 | 932±86 |
| no treatment | - | 0 | 1021±75 | 994±43 |

As is evident from Table 4, administration of Compound A to the mice prior to the X-ray irradiation resulted in the recovery of the erythrocyte count to an almost normal level.

### Experimental Example 4 : Promoting action on recovery of erythrocyte count in mice irradiated with X-rays (therapeutic effects)

BALB/c mice (4-5 weeks old) were exposed to 4.0 Gy total body irradiation. Compound A was intravenously administered for 9 consecutive days starting from the next day of the X-ray irradiation to 9 days thereafter. On day 14 after the X-ray irradiation, blood was taken from orbital vein plexus of mice and erythrocyte count was measured using an automatic cell counter. The experiment was done with 6 mice per group and the results are shown as mean value±standard deviation.

**Table 5**

| Treatment | Test compound | Dose (mg/kg) | Erythrocyte count (10⁴/mm³) |
|---|---|---|---|
| X-irradiation | - | 0 | 327±121 |
| X-irradiation | Comp. A | 0.01 | 535±98 |
| X-irradiation | Comp. A | 0.1 | 686±72 |
| no treatment | - | 0 | 939±69 |

As is evident from Table 5, administration of Compound A to the mice irradiated with X-rays resulted in the recovery of the erythrocyte count to an almost normal level.

### Experimental Example 5 : Promoting action on recovery of erythrocyte count in mice transplanted with bone marrow

ICR mice (6-8 weeks old) were exposed to 8.0 Gy total body irradiation and 10⁷ bone marrow cells from the mice of the same species were transplanted to the mice. Then, Compound A was intravenously administered for 9 consecutive days starting from the next day of the bone marrow transplantation. On day 11 after the bone marrow transplantation, heparinized blood was taken from the abdominal aorta of mice and erythrocyte count was measured using an automatic cell counter. The experiment was done with 5 mice per group.

**Table 6**

| Pretreatment | Test compound | Dose (mg/kg) | Erythrocyte count (10⁴/mm³) |
|---|---|---|---|
| X-irradiation alone | - | 0 | 550±119 |
| X-irradiation+bone marrow transplantation | - | 0 | 637±40 |
| X-irradiation+bone marrow transplantation | Comp. A | 0.01 | 699±51 |
| X-irradiation+bone marrow transplantation | Comp. A | 0.1 | 714±56 |
| no treatment | - | 0 | 722±105 |

As is evident from Table 6, administration of Compound A to the mice transplanted with bone marrow resulted in the recovery of the erythrocyte count to an almost normal level.

Based on the foregoing Experimental Examples, it is evident that administration of Compound A to the animals which were subjected to X-ray irradiation or transplanted with bone marrow results in promoted recovery of decreased erythrocyte count.

### Experimental Example 6 : Stimulating action on phagocytic activity of leukocyte

The experiment was done in accordance with the methods of Stossel et al. [Journal of the Clinical Investigation, vol. 51, p 615 (1972)].

ICR mice (30-35 g body weight) were intraperitoneally administered with glycogen. Two hours later, ascitic leukocytes were taken and a 5 × 10⁶ cell/ml leukocyte suspension was prepared. The compound of the present invention was added to the suspension (200 ml) and 100 µl of mouse serum and 100 µl of dead yeasts (1×10⁸ cell/ml) were added thereto, followed by incubation at 37°C for 20 minutes. Then, about 200 leukocytes in the reaction mixture were observed with a microscope (magnification ×400) and leukocytes which phagocytized one or more dead yeasts were counted. The relative ratio of the count of phagocytic leukocytes when the test compound (0.1 µM) was added thereto to that of the phagocytic leukocytes of the control was calculated and expressed in percentage.

### Experimental Example 7 : Stimulating action on recovery of leukocyte count

ICR mice (20-25 g body weight) were intraperitoneally administered with 200 mg/kg of cyclophosphamide and the compound of the present invention was intravenously administered for 3 days starting from the next day of the administration. On day 4 after the cyclophosphamide administration, the blood of the ICR mice was taken and leukocyte count was measured using Coulter counter. The relative ratio of the count to the peripheral leukocyte count of the mice administered with cyclophosphamide was calculated and expressed in percentage.

**Table 7**

| Pretreatment | Test compound | Dose (mg/kg) | Leukocyte count (10²/mm³) | Increase in leukocyte count (%) |
|---|---|---|---|---|
| cyclophosphamide | - | 0 | 11±4 | 100 |
| cyclophosphamide | Comp. A | 0.01 | 18±8 | 164 |
| cyclophosphamide | Comp. A | 0.1 | 25±6 | 227 |
| cyclophosphamide | Comp. A | 1 | 30±6 | 273 |
| cyclophosphamide | Comp. B | 0.003 | 19±5 | 173 |
| cyclophosphamide | Comp. B | 0.03 | 27±5 | 245 |
| cyclophosphamide | Comp. B | 0.3 | 30±6 | 273 |

As is evident from Table 7, administration of Compound A or Compound B to the mice pretreated with cyclophosphamide resulted in the recovery of the leukocyte count, wherein Compound B exhibited about three times stronger effect than did Compound A.

### Experimental Example 8 : Promoting action on recovery of leukocyte count in mice transplanted with bone marrow

ICR mice (6-8 weeks old) were exposed to 8.0 Gy total body irradiation and 10⁷ bone marrow cells from the mice of the same species were transplanted to the mice. The bone marrow transplantation was performed three times on day 0, day 7 and day 14. Then, the compound of the present invention was intravenously administered for 14 consecutive days starting from the next day of the first bone marrow transplantation. On day 38 after the first bone marrow transplantation, blood was taken and leukocyte count was measured using an automatic cell counter. The experiment was done with 5 mice per group. The relative ratio of the increased leukocyte count to the decreased leukocyte count of the mice transplanted with bone marrow was calculated and expressed in percentage.

**Table 8**

| Treatment | Test compound | Dose (mg/kg) | Leukocyte count (10²/mm³) | Increase in leukocyte count (%) |
|---|---|---|---|---|
| X-irradiation+bone marrow transplantation | - | 0 | 47±6 | 0 |
| X-irradiation+bone marrow transplantation | Comp. A | 0.01 | 65±7 | 30 |
| X-irradiation+bone marrow transplantation | Comp. A | 0.1 | 75±5 | 46 |
| X-irradiation+bone marrow transplantation | Comp. A | 1 | 81±4 | 56 |
| X-irradiation+bone marrow transplantation | Comp. B | 0.01 | 76±10 | 48 |
| X-irradiation+bone marrow transplantation | Comp. B | 0.1 | 89±6 | 69 |
| no treatment | - | 0 | 108±13 | 100 |

As is evident from Table 8, administration of Compound A or Compound B to the bone marrow transplanted mice resulted in the recovery of the leukocyte count, wherein Compound B exhibited about ten times stronger effect than did Compound A.

### Experimental Example 9 : Acute toxicity test

Compound A, Compound B or Compound C was orally administered to rats. As a result, no death case was found at a dose of 1000 mg/kg.

Based on the foregoing Experimental Examples and various pharmacological experiments, it is evident that Compound (1) of the present invention has a stimulating action on recovery of platelet count and erythrocyte count, and is useful as an active ingredient for a pharmaceutical agent which promotes recovery of decreased platelet count and erythrocyte count, caused by drug therapy and radiotherapy for the treatment of malignant tumor.

The S-compound, which is an optically active compound of the Compound (1) of the present invention, has stimulating action on phagocytic activity of leukocyte, stimulating action on phagocytic activity of macrophage, leukocyte count recovery action, stimulating action on resistance to infection, antitumor action, immunological competence-improving action, as well as platelet count recovery action and erythrocyte count recovery action. Accordingly, the compound is useful for the integrated treatment such as potentiation of resistance to infection and alleviation of bone marrow disorders in the treatment of infectious diseases and malignant tumor. In addition, the compound is useful for the treatment of various diseases caused by myelosuppression, such as aplastic anemia, myelodysplastic syndromes, myelogeneous anemia, congenital anemia, renal anemia, congenital and idiopathic neutropenia and idiopathic thrombocytopenic purpura. Particularly, the S-compound, which is an optically active compound, exhibits superior action as recited above, in comparison with the corresponding racemate and R-compound.

The pharmaceutical preparation of the present invention preferably contains a therapeutically effective amount of the compound, and said phamaceutical preparation may be prepared by admixing the active ingredient with pharmaceutically acceptable carriers, excipients, diluents, solubilizers, disintegrators, binders etc. and formulating the admixture into tablets, powders, capsules, injections, suppositories or infusions which can be safely administered orally or parenterally to mammals such as human. The dose is generally 2.5-100 mg for oral administration and 0.5-20 mg for intravenous administration to an adult per day.

The (S)-3-[4-(2-dimethylamino-1-methylethoxy)phenyl]-1H-pyrazolo[3,4-b]pyridine-1-acetic acid ethyl ester obtainable by the present invention is quickly de-ethyl esterified in the body and converted to an active compound, (S)-3-[4-(2-dimethylamino-1-methylethoxy)phenyl]-1H-pyrazolo[3,4-b]pyridine-1-acetic acid, which is conducive to superior absorption by oral administration and superior bioavailability, thus making its use as an active ingredient for oral preparations beneficial.

The present invention is described in more detail by illustrating Examples and Formulation Examples in the following, to which the invention is not limited.

### Example 1

(1) 60% Sodium hydride (27 g) was suspended in toluene (150 ml) and a mixture of (S)-1-dimethylamino-2-propanol (75 g) in toluene (200 ml) was dropwise added thereto at 50-60°C. The mixture was cooled and dimethylformamide (200 ml) was added thereto. Then, 2-chloro-3-(4-fluorobenzoyl)pyridine (182 g) was added in several times divided portions at -10 - -5°C. The temperature was gradually raised to 40°C over 3 hours and the mixture was stirred at 40°C for 2 hours to terminate the reaction. Water was added thereto, and toluene layer was partitioned and washed with water. The toluene layer was extracted with dilute hydrochloric acid. The extract was made alkaline with potassium carbonate and the liberated oily substance was extracted with toluene. The toluene was distilled away to give 190 g of crude (S)-2-chloro-3-[4-(2-dimethylamino-1-methylethoxy)benzoyl]pyridine as an oily substance.
(2) The crude product (190 g), pyridine (600 ml) and hydrazine hydrate (92 g) were refluxed with stirring for 12 hours. The pyridine was distilled away and toluene (500 ml) was added to the residue. The mixture was heated to 40-50°C for dissolution. The mixture was washed with warm water and the toluene was distilled away. Isopropyl ether was added to the residue to allow crystallization. After collection by filtration, the crystals were recrystallized from the mixed solution of isopropyl ether and isopropyl alcohol to give 100.7 g of (S)-3-[4-(2-dimethylamino-1-methylethoxy)phenyl]-1H-pyrazolo[3,4-b]pyridine, melting point 125-127°C. [α]_{D}²³ = +15.3° (1%, methanol)
(3) 60% Sodium hydride (2.7 g) was suspended in toluene (46 ml) and dimethylformamide (31 ml) was added thereto under ice-cooling. Then, (S)-3-[4-(2-dimethylamino-1-methylethoxy)phenyl]-1H-pyrazolo[3,4-b]pyridine (18 g) was added thereto in 2 or 3 times divided portions. After stirring the mixture for 15 minutes, a mixed solution of ethyl chloroacetate (8.2 g) and toluene (16 ml) was dropwise added thereto, and the mixture was allowed to react at room temperature for 1 hour and at 40°C for 1 hour. After cooling, water was added thereto and a toluene layer was partitioned. The layer was washed with water and dried. The toluene was distilled away to give 20.8 g of crude ethyl (S)-3-[4-(2-dimethylamino-1-methylethoxy)phenyl]-1H-pyrazolo[3,4-b]pyridine-1-acetate as an oily substance.
(4) The crude ethyl (S)-3-[4-(2-dimethylamino-1-methylethoxy)phenyl]-1H-pyrazolo[3,4-b]pyridine-1-acetate (20.8 g) was dissolved in ethyl acetate (150 ml) and a 15% hydrochloric acid - ethanol solution (13 g) was added thereto to give hydrochloride. The resulting crystals were purified from a mixed solution of ethyl acetate - ethanol to give 17.2 g of ethyl (S)-3-[4-(2-dimethylamino-1-methylethoxy)phenyl]-1H-pyrazolo[3,4-b]pyridine-1-acetate hydrochloride, melting point 165-166°C. [α]_{D}²⁵ = +16.1° (1%, ethanol)

### Example 2

The crude product (20.8 g) from Example 1(3) above was dissolved in methanol (43 ml) and a solution of sodium hydroxide (2.4 g) in water (21 ml) was added thereto. The mixture was stirred at room temperature for 1 hour. The pH thereof was adjusted to 7 with dilute hydrochloric acid and methanol was distilled away. The residue was purified by the use of Diaion PH-20 (trade mark). After recrystallization (inclusive of crystal water) from aqueous isopropyl alcohol, ethanol was added and the mixture was heated to give (S)-3-[4-(2-dimethylamino-1-methylethoxy)phenyl]-1H-pyrazolo[3,4-b]pyridine-1-acetic acid, melting point 212-213°C. [α]_{D}²² = +25.7° (1%, methanol)

### Example 3

(1) (R)-1-Dimethylamino-2-propanol was reacted in the same manner as in Example 1 (1) and (2) to give (R)-3-[4-(2-dimethylamino-1-methylethoxy)phenyl]-1H-pyrazolo[3,4-b]pyridine, melting point 125-127°C. [α]_{D}²³ = -15.0° (1%, methanol)
(2) (R)-3-[4-(2-Dimethylamino-1-methylethoxy)phenyl]-1H-pyrazolo[3,4-b]pyridine was reacted in the same manner as in Example 1 (3) and Example 2 to give (R)-3-[4-(2-dimethylamino-1-methylethoxy)phenyl]-1H-pyrazolo[3,4-b]pyridine-1-acetic acid, melting point 212-214°C. [α]_{D}²³ = -25.0° (1%, methanol)

### Example 4

(1) 60% Sodium hydride (4 g) was suspended in toluene (30 ml) and thereto was dropwise added a solution of 3-dimethylaminopropanol (15 g) and toluene (20 ml) to adjust alcoholate. The mixture was cooled and added with dimethylformamide (50 ml) and then with 2-chloro-3-(4-fluorobenzoyl)-6-isopropylpyridine (30 g) at -10 - -5°C. The temperature was gradually raised to 40°C over 3 hours and the mixture was heated at 40°C and stirred at said temperature for 1 hour to terminate the reaction. Water was added thereto and a toluene layer was partitioned and washed with water. The toluene layer was extracted with dilute hydrochloric acid and made alkaline with potassium carbonate. The liberated oily substance was extracted with toluene. The toluene in the extract was distilled away to give 24 g of crude 2-chloro-3-[4-(3-dimethylaminopropoxy)benzoyl]-6-isopropylpyridine as an oily substance.
   The crude product (24 g), pyridine (100 ml) and hydrazine hydrate (16.6 g) were refluxed with stirring for 12 hours. The pyridine was distilled away and the residue was dissolved in toluene. The mixture was washed with a solution of dilute sodium hydroxide and water and the toluene was distilled away. The resulting crystals were purified with isopropyl alcohol to give 15.3 g of 3-[4-(3-dimethylaminopropoxy)phenyl]-6-isopropyl-1H-pyrazolo[3,4-b]pyridine, melting point 153-155°C.
(2) 60% Sodium hydride (0.76 g) was suspended in toluene (10 ml), and dimethylformamide (20 ml) and 3-[4-(3-dimethylaminopropoxy)phenyl]-6-isopropyl-1H-pyrazolo[3,4-b]pyridine (5.8 g) were added thereto under ice-cooling. After stirring the mixture for 15 minutes, a solution of ethyl chloroacetate (2.3 g) in toluene (10 ml) was dropwise added thereto and the mixture was allowed to react at room temperature for 1 hour and at 40°C for 1 hour. After cooling, water was added thereto, and the toluene layer was partitioned, washed with water and dried. The toluene was distilled away and the resulting oily substance was purified by silica gel column chromatography to give 4.6 g of ethyl 3-[4-(3-dimethylaminopropoxy)phenyl]-6-isopropyl-1H-pyrazolo[3,4-b]pyridine-1-acetate as an oily substance.
   ¹H-NMR (100MHz, CDCl₃) δ: 1.26 (t,3H), 1.33 (d,6H), 1.82-2.04 (m,2H), 2.26 (s,6H), 2.47 (t,2H), 3.00-3.29 (m,1H), 4.08 (t,2H), 4.22 (q,2H), 5.30 (s,2H), 7.00 (d,2H), 7.04 (d,1H), 7.85 (d,2H), 8.16 (d,1H)

### Example 5

Ethyl 3-[4-(3-dimethylaminopropoxy)phenyl]-6-isopropyl-1H-pyrazolo[3,4-b]pyridine-1-acetate (4.5 g), water (20 ml) and triethylamine (2.1 g) were refluxed with stirring for 18 hours. Water was distilled away under reduced pressure and ethanol was added to the residue. The mixture was concentrated to dryness under reduced pressure to give crystals. The crystals were purified with methanol to give 3.0 g of 3-[4-(3-dimethylaminopropoxy)phenyl]-6-isopropyl-1H-pyrazolo[3,4-b]pyridine-1-acetic acid, melting point 202-204°C.

### Example 6

1-Dimethylamino-2-propanol and 2-chloro-3-(4-fluorobenzoyl)-6-isopropylpyridine were reacted and treated in the same manner as in Example 4 (1) and (2) to give ethyl 3-[4-(2-dimethylamino-1-methylethoxy)phenyl]-6-isopropyl-1H-pyrazolo[3,4-b]pyridine-1-acetate.

¹H-NMR (CDCl₃) δ: 1.18-1.40 (m,12H), 2.32 (s,6H), 2.44-2.90 (m,2H), 2.92-3.31 (m,1H), 4.20 (q,2H), 4.54 (dd,1H), 5.27 (s,2H), 6.97 (d,2H), 7.01 (d,1H), 7.78 (d,2H), 8.12 (d,1H)

### Example 7

Ethyl 3-[4-(2-dimethylamino-1-methylethoxy)phenyl]-6-isopropyl-1H-pyrazolo[3,4-b]pyridine-1-acetate obtained in Example 6 was reacted and treated in the same manner as in Example 5 to give 3-[4-(2-dimethylamino-1-methylethoxy)phenyl]-6-isopropyl-1H-pyrazolo[3,4-b]pyridine-1-acetic acid.

¹H-NMR (CDCl₃) δ: 1.18-1.38 (m,9H), 2.32 (s,6H), 2.86-3.30 (m,3H), 4.75 (dd,1H), 5.17 (s,2H), 6.91 (d,2H), 6.96 (d,1H), 7.79 (d,2H), 8.07 (d,1H)

### Example 8

(S)-1-Dimethylamino-2-propanol and 2-chloro-3-(4-fluorobenzoyl)-6-isopropylpyridine are reacted and treated in the same manner as in Example 1 (1)-(4) to give ethyl (S)-3-[4-(2-dimethylamino-1-methylethoxy)phenyl]-6-isopropyl-1H-pyrazolo[3,4-b]pyridine-1-acetate.

The ethyl ester compound obtained above is reacted and treated in the same manner as in Example 2 to give (S)-3-[4-(2-dimethylamino-1-methylethoxy)phenyl]-6-isopropyl-1H-pyrazolo[3,4-b]pyridine-1-acetic acid.

### Formulation Example 1

Compound A (10 g) and sodium chloride (9 g) were dissolved in injectable water (10 ℓ). After sterilizing by filtration, 10 ml therefrom was charged in an ampoule, which was sealed and subjected to high-pressure steam sterilization to give an injection.

### Formulation Example 2

Using Compound B, an injection containing Compound B is prepared in the same manner as in Formulation Example 1.

### Formulation Example 3

Compound C (50 mg) was thoroughly kneaded in a kneader with lactose (98 mg), corn starch (45 mg) and hydroxypropylcellulose (3 mg). The kneaded product was passed through a 200-mesh sieve, dried at 50°C and passed through a 24-mesh sieve. The resulting mixture was mixed with talc (3 mg) and magnesium stearate (1 mg) and prepared into tablets weighing 200 mg per tablet with the use of a 9 mm diameter punch. The tablets may be sugar or film coated as necessary.

The present invention has been described in detail in the specification including Examples. The present invention and particularly Examples are subject to various changes and modifications insofar as they are within the spirit and scope of the present invention.

## Claims

1. An agent for treating thrombocytopenia, comprising, as an active ingredient, a fused pyrazole compound of the formula 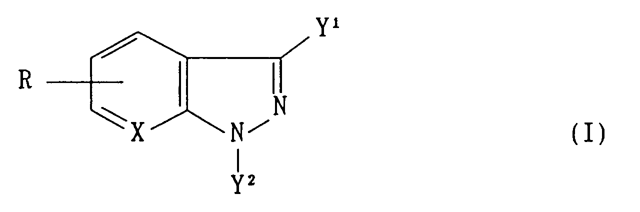 wherein
X is =N- or =CH-;
R is a hydrogen, an alkyl, an alkoxy or a halogen;
Y¹ is a group of the formula 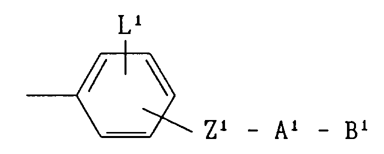 or
a phenyl which may be substituted by 1 to 3 substituent(s) selected from the group consisting of halogen, nitro, amino, alkyl and alkoxy; and
Y² is a group of the formula 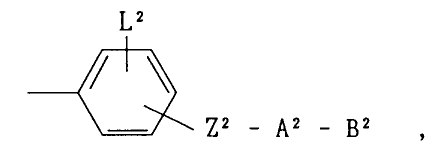 a group of the formula : -A²-B², a hydrogen, an alkyl, a phenylalkyl wherein phenyl nucleus may be substituted by 1 to 3 substituent(s) selected from the group consisting of halogen, nitro, amino, alkyl and alkoxy, a phenyl which may be substituted by 1 to 3 substituent(s) selected from the group consisting of halogen, nitro, amino, alkyl and alkoxy, an alkoxyalkyl, a hydroxyalkyl, an alkoxycarbonylalkyl, a carboxyalkyl or an acyloxyalkyl;
provided that either or both of Y¹ and Y² is(are) represented by the formula 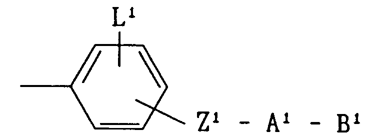 or the formula 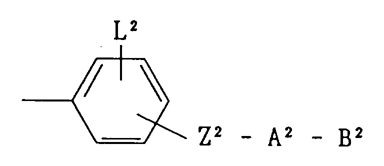 wherein Z¹ and Z² are the same or different and each is -O-, -S-or -NR³- wherein R³ is a hydrogen or an alkyl, A¹ and A² are the same or different and each is an alkylene, B¹ and B² are the same or different and each is a carboxyl, an alkoxycarbonyl, a phenylalkoxycarbonyl wherein phenyl nucleus may be substituted by 1 to 3 substituent(s) selected from the group consisting of halogen, nitro, amino, alkyl and alkoxy, an acyl, a hydroxyl, an acyloxy or a group of the formula 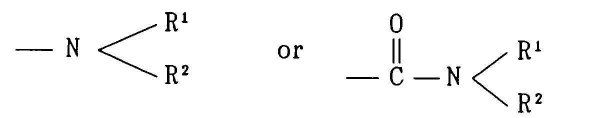 wherein R¹ and R² are the same or different and each is a hydrogen, an alkyl, a dialkylaminoalkyl or a hydroxyalkyl, or R¹ and R² may form a heterocyclic ring together with the adjoining nitrogen atom, and L¹ and L² are the same or different and each is a hydrogen, a halogen, an alkyl or an alkoxy, an optical isomer thereof or a pharmaceutically acceptable salt of said compound or the isomer.

2. An agent for treating thrombocytopenia, comprising, as an active ingredient, a fused pyrazole compound of the formula 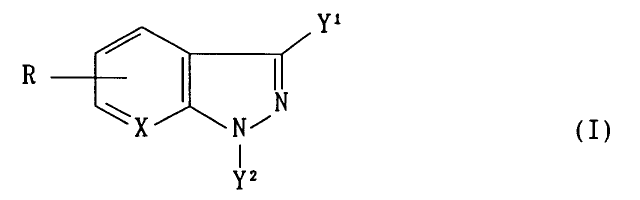 wherein
X is =N-;
R is a hydrogen, an alkyl, an alkoxy or a halogen;
Y¹ is a group of the formula 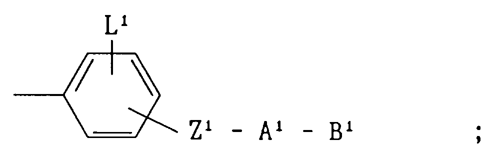 and
Y² is a group of the formula : -A²-B² or a hydrogen;
wherein Z¹ is -O-, A¹ and A² are the same or different and each is an alkylene, B¹ is a group of the formula 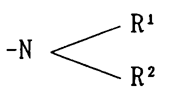 wherein R¹ and R² are the same or different and each is a hydrogen, an alkyl, a dialkylaminoalkyl or hydroxyalkyl, or R¹ and R² may form a heterocyclic ring together with the adjoining nitrogen atom, B² is a carboxyl, an alkoxycarbonyl or a phenylalkoxycarbonyl wherein phenyl nucleus may be substituted by 1 to 3 substituent(s) selected from the group consisting of halogen, nitro, amino, alkyl and alkoxy and L¹ is a hydrogen, a halogen, an alkyl or an alkoxy, an optical isomer thereof or a pharmaceutically acceptable salt of said compound or the isomer.

3. An agent for treating thrombocytopenia, comprising, as an active ingredient, a compound selected from the group consisting of
3-[4-(2-dimethylamino-1-methylethoxy)phenyl]-1H-pyrazolo[3,4-b]pyridine-1-acetic acid,
(S)-3-[4-(2-dimethylamino-1-methylethoxy)phenyl]-1H-pyrazolo[3,4-b]pyridine-1-acetic acid,
ethyl 3-[4-(2-dimethylamino-1-methylethoxy)phenyl]-1H-pyrazolo[3,4-b]pyridine-1-acetate,
ethyl (S)-3-[4-(2-dimethylamino-1-methylethoxy)phenyl]-1H-pyrazolo[3,4-b]pyridine-1-acetate,
3-[4-(3-dimethylaminopropoxy)phenyl]-6-isopropyl-1H-pyrazolo[3,4-b]pyridine-1-acetic acid,
ethyl 3-[4-(3-dimethylaminopropoxy)phenyl]-6-isopropyl-1H-pyrazolo[3,4-b]pyridine-1-acetate,
3-[4-(2-dimethylamino-1-methylethoxy)phenyl]-6-isopropyl-1H-pyrazolo[3,4-b]pyridine-1-acetic acid,
(S)-3-[4-(2-dimethylamino-1-methylethoxy)phenyl]-6-isopropyl-1H-pyrazolo[3,4-b]pyridine-1-acetic acid,
ethyl 3-[4-(2-dimethylamino-1-methylethoxy)phenyl]-6-isopropyl-1H-pyrazolo[3,4-b]pyridine-1-acetate,
ethyl (S)-3-[4-(2-dimethylamino-1-methylethoxy)phenyl]-6-isopropyl-1H-pyrazolo[3,4-b]pyridine-1-acetate,
6-methyl-3-[4-(3-dimethylaminopropoxy)phenyl]-1H-pyrazolo[3,4-b]pyridine-1-acetic acid and
ethyl 6-methyl-3-[4-(3-dimethylaminopropoxy)phenyl]-1H-pyrazolo[3,4-b]pyridine-1-acetate, or a pharmaceutically acceptable salt thereof.

4. A method for treating thrombocytopenia, comprising administering a fused pyrazole compound of the formula 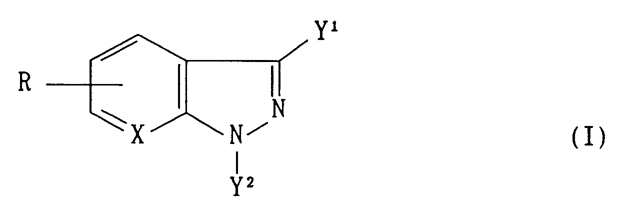 wherein
X is =N- or =CH-;
R is a hydrogen, an alkyl, an alkoxy or a halogen;
Y¹ is a group of the formula 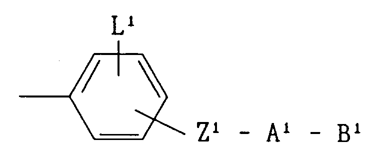 or
a phenyl which may be substituted by 1 to 3 substituent(s) selected from the group consisting of halogen, nitro, amino, alkyl and alkoxy; and
Y² is a group of the formula 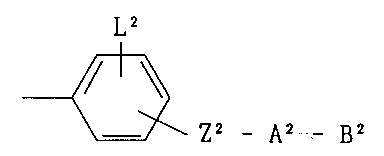 a group of the formula : -A²-B², a hydrogen, an alkyl, a phenylalkyl wherein phenyl nucleus may be substituted by 1 to 3 substituent(s) selected from the group consisting of halogen, nitro, amino, alkyl and alkoxy, a phenyl which may be substituted by 1 to 3 substituent(s) selected from the group consisting of halogen, nitro, amino, alkyl and alkoxy, an alkoxyalkyl, a hydroxyalkyl, an alkoxycarbonylalkyl, a carboxyalkyl or an acyloxyalkyl;
provided that either or both of Y¹ and Y² is(are) represented by the formula 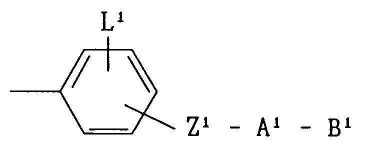 or the formula 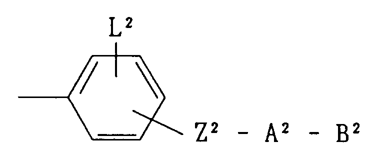 wherein Z¹ and Z² are the same or different and each is -O-, -S-or -NR³- wherein R³ is a hydrogen or an alkyl, A¹ and A² are the same or different and each is an alkylene, B¹ and B² are the same or different and each is a carboxyl, an alkoxycarbonyl, a phenylalkoxycarbonyl wherein phenyl nucleus may be substituted by 1 to 3 substituent(s) selected from the group consisting of halogen, nitro, amino, alkyl and alkoxy, an acyl, a hydroxyl, an acyloxy or a group of the formula 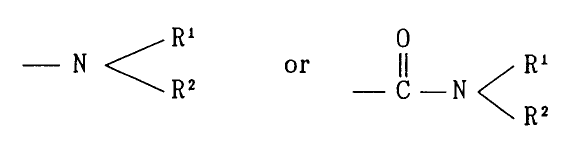 wherein R¹ and R² are the same or different and each is a hydrogen, an alkyl, a dialkylaminoalkyl or a hydroxyalkyl, or R¹ and R² may form a heterocyclic ring together with the adjoining nitrogen atom, and L¹ and L² are the same or different and each is a hydrogen, a halogen, an alkyl or an alkoxy, an optical isomer thereof or a pharmaceutically acceptable salt of said compound or the isomer.

5. A method for treating thrombocytopenia, comprising administering a fused pyrazole compound of the formula 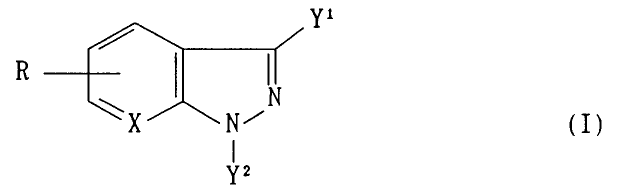 wherein
X is =N-;
R is a hydrogen, an alkyl, an alkoxy or a halogen;
Y¹ is a group of the formula 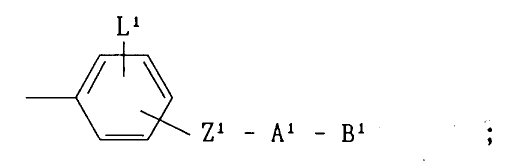 and
Y² is a group of the formula : -A²-B² or a hydrogen;
wherein Z¹ is -O-, A¹ and A² are the same or different and each is an alkylene, B¹ is a group of the formula 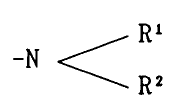 wherein R¹ and R² are the same or different and each is a hydrogen, an alkyl, a dialkylaminoalkyl or hydroxyalkyl, or R¹ and R² may form a heterocyclic ring together with the adjoining nitrogen atom, B² is a carboxyl, an alkoxycarbonyl, a phenylalkoxycarbonyl wherein phenyl nucleus may be substituted by 1 to 3 substituent(s) selected from the group consisting of halogen, nitro, amino, alkyl and alkoxy and L¹ is a hydrogen, a halogen, an alkyl or an alkoxy, an optical isomer thereof or a pharmaceutically acceptable salt of said compound or the isomer.

6. A method for treating thrombocytopenia, comprising administering a compound selected from the group consisting of
3-[4-(2-dimethylamino-1-methylethoxy)phenyl]-1H-pyrazolo[3,4-b]pyridine-1-acetic acid,
(S)-3-[4-(2-dimethylamino-1-methylethoxy)phenyl]-1H-pyrazolo[3,4-b]pyridine-1-acetic acid,
ethyl 3-[4-(2-dimethylamino-1-methylethoxy)phenyl]-1H-pyrazolo[3,4-b]pyridine-1-acetate,
ethyl (S)-3-[4-(2-dimethylamino-1-methylethoxy)phenyl]-1H-pyrazolo[3,4-b]pyridine-1-acetate,
3-[4-(3-dimethylaminopropoxy)phenyl]-6-isopropyl-1H-pyrazolo[3,4-b]pyridine-1-acetic acid,
ethyl 3-[4-(3-dimethylaminopropoxy)phenyl]-6-isopropyl-1H-pyrazolo[3,4-b]pyridine-1-acetate,
3-[4-(2-dimethylamino-1-methylethoxy)phenyl]-6-isopropyl-1H-pyrazolo[3,4-b]pyridine-1-acetic acid,
(S)-3-[4-(2-dimethylamino-1-methylethoxy)phenyl]-6-isopropyl-1H-pyrazolo[3,4-b]pyridine-1-acetic acid,
ethyl 3-[4-(2-dimethylamino-1-methylethoxy)phenyl]-6-isopropyl-1H-pyrazolo[3,4-b]pyridine-1-acetate,
ethyl (S)-3-[4-(2-dimethylamino-1-methylethoxy)phenyl]-6-isopropyl-1H-pyrazolo[3,4-b]pyridine-1-acetate,
6-methyl-3-[4-(3-dimethylaminopropoxy)phenyl]-1H-pyrazolo[3,4-b]pyridine-1-acetic acid and
ethyl 6-methyl-3-[4-(3-dimethylaminopropoxy)phenyl]-1H-pyrazolo[3,4-b]pyridine-1-acetate, or a pharmaceutically acceptable salt thereof.

7. A use of a fused pyrazole compound of the formula 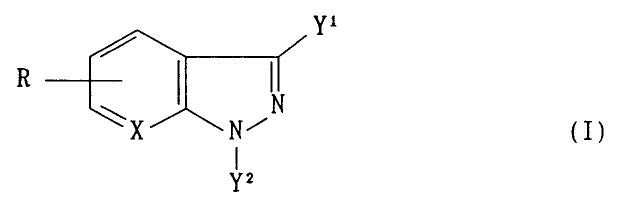 wherein
X is =N- or =CH-;
R is a hydrogen, an alkyl, an alkoxy or a halogen;
Y¹ is a group of the formula 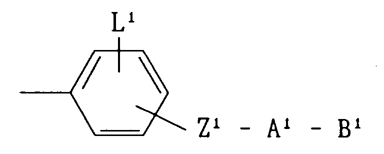 or
a phenyl which may be substituted by 1 to 3 substituent(s) selected from the group consisting of halogen, nitro, amino, alkyl and alkoxy; and
Y² is a group of the formula 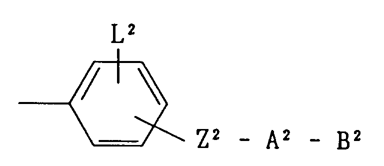 a group of the formula : -A²-B², a hydrogen, an alkyl, a phenylalkyl wherein phenyl nucleus may be substituted by 1 to 3 substituent(s) selected from the group consisting of halogen, nitro, amino, alkyl and alkoxy, a phenyl which may be substituted by 1 to 3 substituent(s) selected from the group consisting of halogen, nitro, amino, alkyl and alkoxy, an alkoxyalkyl, a hydroxyalkyl, an alkoxycarbonylalkyl, a carboxyalkyl or an acyloxyalkyl;
provided that either or both of Y¹ and Y² is(are) represented by the formula 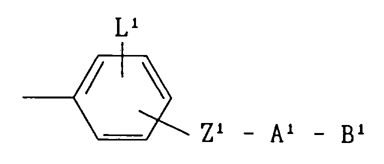 or the formula 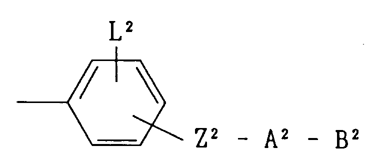 wherein Z¹ and Z² are the same or different and each is -O-, -S-or -NR³- wherein R³ is a hydrogen or an alkyl, A¹ and A² are the same or different and each is an alkylene, B¹ and B² are the same or different and each is a carboxyl, an alkoxycarbonyl, a phenylalkoxycarbonyl wherein phenyl nucleus may be substituted by 1 to 3 substituent(s) selected from the group consisting of halogen, nitro, amino, alkyl and alkoxy, an acyl, a hydroxyl, an acyloxy or a group of the formula 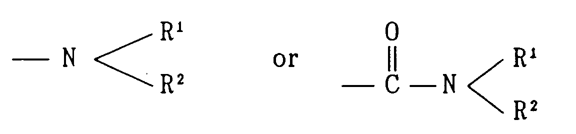 wherein R¹ and R² are the same or different and each is a hydrogen, an alkyl, a dialkylaminoalkyl or a hydroxyalkyl, or R¹ and R² may form a heterocyclic ring together with the adjoining nitrogen atom, and L¹ and L² are the same or different and each is a hydrogen, a halogen, an alkyl or an alkoxy, an optical isomer thereof or a pharmaceutically acceptable salt of said compound or the isomer for the production of an agent for treating thrombocytopenia.

8. A use of a fused pyrazole compound of the formula 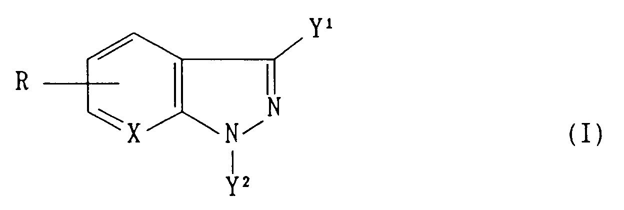 wherein
X is =N-;
R is a hydrogen, an alkyl, an alkoxy or a halogen;
Y¹ is a group of the formula 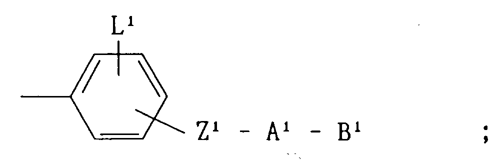 and
Y² is a group of the formula : -A²-B² or a hydrogen;
wherein Z¹ is -O-, A¹ and A² are the same or different and each is an alkylene, B¹ is a group of the formula 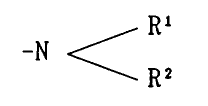 wherein R¹ and R² are the same or different and each is a hydrogen, an alkyl, a dialkylaminoalkyl or hydroxyalkyl, or R¹ and R² may form a heterocyclic ring together with the adjoining nitrogen atom, B² is a carboxyl, an alkoxycarbonyl or a phenylalkoxycarbonyl wherein phenyl nucleus may be substituted by 1 to 3 substituent(s) selected from the group consisting of halogen, nitro, amino, alkyl and alkoxy and L¹ is a hydrogen, a halogen, an alkyl or an alkoxy, an optical isomer thereof or a pharmaceutically acceptable salt of said compound or the isomer for the production of an agent for treating thrombocytopenia.

9. A use of a compound selected from the group consisting of
3-[4-(2-dimethylamino-1-methylethoxy)phenyl]-1H-pyrazolo[3,4-b]pyridine-1-acetic acid,
(S)-3-[4-(2-dimethylamino-1-methylethoxy)phenyl]-1H-pyrazolo[3,4-b]pyridine-1-acetic acid,
ethyl 3-[4-(2-dimethylamino-1-methylethoxy)phenyl]-1H-pyrazolo[3,4-b]pyridine-1-acetate,
ethyl (S)-3-[4-(2-dimethylamino-1-methylethoxy)phenyl]-1H-pyrazolo[3,4-b]pyridine-1-acetate,
3-[4-(3-dimethylaminopropoxy)phenyl]-6-isopropyl-1H-pyrazolo[3,4-b]pyridine-1-acetic acid,
ethyl 3-[4-(3-dimethylaminopropoxy)phenyl]-6-isopropyl-1H-pyrazolo[3,4-b]pyridine-1-acetate,
3-[4-(2-dimethylamino-1-methylethoxy)phenyl]-6-isopropyl-1H-pyrazolo[3,4-b]pyridine-1-acetic acid,
(S)-3-[4-(2-dimethylamino-1-methylethoxy)phenyl]-6-isopropyl-1H-pyrazolo[3,4-b]pyridine-1-acetic acid,
ethyl 3-[4-(2-dimethylamino-1-methylethoxy)phenyl]-6-isopropyl-1H-pyrazolo[3,4-b]pyridine-1-acetate,
ethyl (S)-3-[4-(2-dimethylamino-1-methylethoxy)phenyl]-6-isopropyl-1H-pyrazolo[3,4-b]pyridine-1-acetate,
6-methyl-3-[4-(3-dimethylaminopropoxy)phenyl]-1H-pyrazolo[3,4-b]pyridine-1-acetic acid and
ethyl 6-methyl-3-[4-(3-dimethylaminopropoxy)phenyl]-1H-pyrazolo[3,4-b]pyridine-1-acetate, or a pharmaceutically acceptable salt thereof for the production of an agent for treating thrombocytopenia.

10. An agent for treating erythropenia, comprising, as an active ingredient, a fused pyrazole compound of the formula 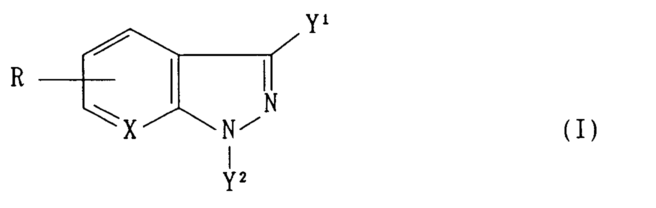 wherein
X is =N- or =CH-;
R is a hydrogen, an alkyl, an alkoxy or a halogen;
Y¹ is a group of the formula 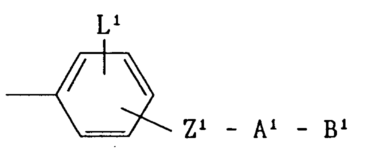 or
a phenyl which may be substituted by 1 to 3 substituent(s) selected from the group consisting of halogen, nitro, amino, alkyl and alkoxy; and
Y² is a group of the formula 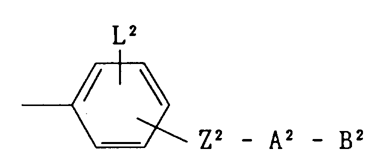 a group of the formula : -A²-B², a hydrogen, an alkyl, a phenylalkyl wherein phenyl nucleus may be substituted by 1 to 3 substituent(s) selected from the group consisting of halogen, nitro, amino, alkyl and alkoxy, a phenyl which may be substituted by 1 to 3 substituent(s) selected from the group consisting of halogen, nitro, amino, alkyl and alkoxy, an alkoxyalkyl, a hydroxyalkyl, an alkoxycarbonylalkyl, a carboxyalkyl or an acyloxyalkyl;
provided that either or both of Y¹ and Y² is(are) represented by the formula 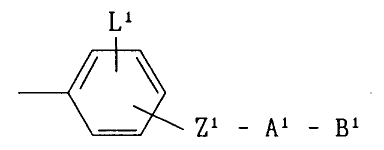 or the formula 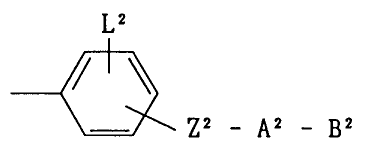 wherein Z¹ and Z² are the same or different and each is -O-, -S-or -NR³- wherein R³ is a hydrogen or an alkyl, A¹ and A² are the same or different and each is an alkylene, B¹ and B² are the same or different and each is a carboxyl, an alkoxycarbonyl, a phenylalkoxycarbonyl wherein phenyl nucleus may be substituted by 1 to 3 substituent(s) selected from the group consisting of halogen, nitro, amino, alkyl and alkoxy, an acyl, a hydroxyl, an acyloxy or a group of the formula 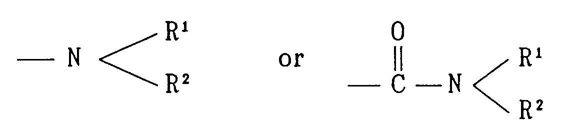 wherein R¹ and R² are the same or different and each is a hydrogen, an alkyl, a dialkylaminoalkyl or a hydroxyalkyl, or R¹ and R² may form a heterocyclic ring together with the adjoining nitrogen atom, and L¹ and L² are the same or different and each is a hydrogen, a halogen, an alkyl or an alkoxy, an optical isomer thereof or a pharmaceutically acceptable salt of said compound or the isomer.

11. An agent for treating erythropenia, comprising, as an active ingredient, a fused pyrazole compound of the formula 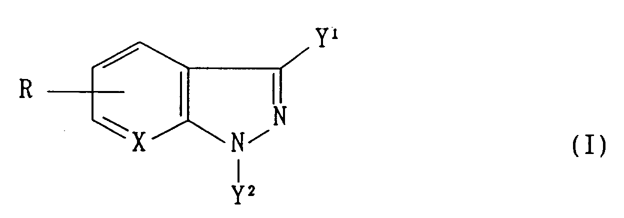 wherein
X is =N-;
R is a hydrogen, an alkyl, an alkoxy or a halogen;
Y¹ is a group of the formula 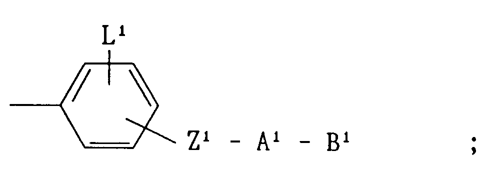 and
Y² is a group of the formula : -A²-B² or a hydrogen;
wherein Z¹ is -O-, A¹ and A² are the same or different and each is an alkylene, B¹ is a group of the formula 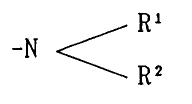 wherein R¹ and R² are the same or different and each is a hydrogen, an alkyl, a dialkylaminoalkyl or hydroxyalkyl, or R¹ and R² may form a heterocyclic ring together with the adjoining nitrogen atom, B² is a carboxyl, an alkoxycarbonyl or a phenylalkoxycarbonyl wherein phenyl nucleus may be substituted by 1 to 3 substituent(s) selected from the group consisting of halogen, nitro, amino, alkyl and alkoxy) and L¹ is a hydrogen, a halogen, an alkyl or an alkoxy, an optical isomer thereof or a pharmaceutically acceptable salt of said compound or the isomer.

12. An agent for treating erythropenia, comprising, as an active ingredient, a compound selected from the group consisting of
3-[4-(2-dimethylamino-1-methylethoxy)phenyl]-1H-pyrazolo[3,4-b]pyridine-1-acetic acid,
(S)-3-[4-(2-dimethylamino-1-methylethoxy)phenyl]-1H-pyrazolo[3,4-b]pyridine-1-acetic acid,
ethyl 3-[4-(2-dimethylamino-1-methylethoxy)phenyl]-1H-pyrazolo[3,4-b]pyridine-1-acetate,
ethyl (S)-3-[4-(2-dimethylamino-1-methylethoxy)phenyl]-1H-pyrazolo[3,4-b]pyridine-1-acetate,
3-[4-(3-dimethylaminopropoxy)phenyl]-6-isopropyl-1H-pyrazolo[3,4-b]pyridine-1-acetic acid,
ethyl 3-[4-(3-dimethylaminopropoxy)phenyl]-6-isopropyl-1H-pyrazolo[3,4-b]pyridine-1-acetate,
3-[4-(2-dimethylamino-1-methylethoxy)phenyl]-6-isopropyl-1H-pyrazolo[3,4-b]pyridine-1-acetic acid,
(S)-3-[4-(2-dimethylamino-1-methylethoxy)phenyl]-6-isopropyl-1H-pyrazolo[3,4-b]pyridine-1-acetic acid,
ethyl 3-[4-(2-dimethylamino-1-methylethoxy)phenyl]-6-isopropyl-1H-pyrazolo[3,4-b)pyridine-1-acetate,
ethyl (S)-3-[4-(2-dimethylamino-1-methylethoxy)phenyl]-6-isopropyl-1H-pyrazolo[3,4-b]pyridine-1-acetate,
6-methyl-3-[4-(3-dimethylaminopropoxy)phenyl]-1H-pyrazolo[3,4-b]pyridine-1-acetic acid and
ethyl 6-methyl-3-[4-(3-dimethylaminopropoxy)phenyl]-1H-pyrazolo[3,4-b]pyridine-1-acetate, or a pharmaceutically acceptable salt thereof.

13. A method for treating erythropenia, comprising administering a fused pyrazole compound of the formula 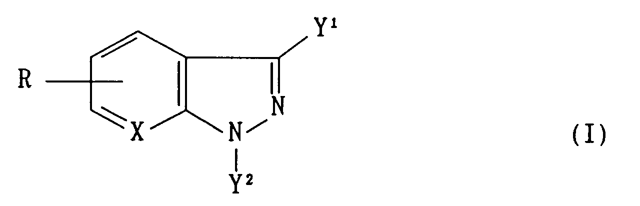 wherein
X is =N- or =CH-;
R is a hydrogen, an alkyl, an alkoxy or a halogen;
Y¹ is a group of the formula 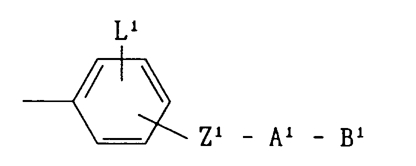 or
a phenyl which may be substituted by 1 to 3 substituent(s) selected from the group consisting of halogen, nitro, amino, alkyl and alkoxy; and
Y² is a group of the formula 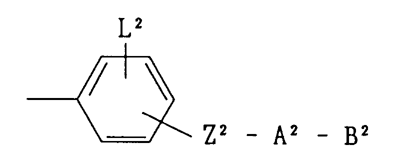 a group of the formula : -A²-B², a hydrogen, an alkyl, a phenylalkyl wherein phenyl nucleus may be substituted by 1 to 3 substituent(s) selected from the group consisting of halogen, nitro, amino, alkyl and alkoxy, a phenyl which may be substituted by 1 to 3 substituent(s) selected from the group consisting of halogen, nitro, amino, alkyl and alkoxy, an alkoxyalkyl, a hydroxyalkyl, an alkoxycarbonylalkyl, a carboxyalkyl or an acyloxyalkyl;
provided that either or both of Y¹ and Y² is(are) represented by the formula 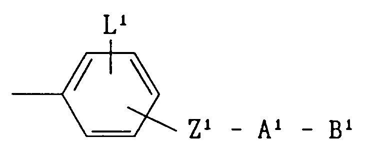 or the formula 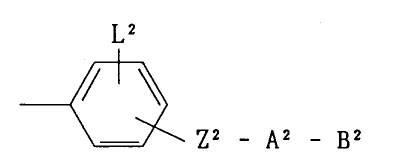 wherein Z¹ and Z² are the same or different and each is -O-, -S-or -NR³- (wherein R³ is a hydrogen or an alkyl), A¹ and A² are the same or different and each is an alkylene, B¹ and B² are the same or different and each is a carboxyl, an alkoxycarbonyl, a phenylalkoxycarbonyl wherein phenyl nucleus may be substituted by 1 to 3 substituent(s) selected from the group consisting of halogen, nitro, amino, alkyl and alkoxy, an acyl, a hydroxyl, an acyloxy or a group of the formula 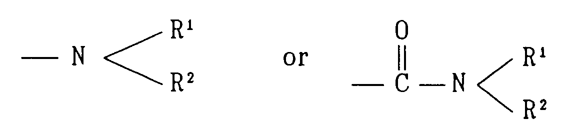 wherein R¹ and R² are the same or different and each is a hydrogen, an alkyl, a dialkylaminoalkyl or a hydroxyalkyl, or R¹ and R² may form a heterocyclic ring together with the adjoining nitrogen atom, and L¹ and L² are the same or different and each is a hydrogen, a halogen, an alkyl or an alkoxy, an optical isomer thereof or a pharmaceutically acceptable salt of said compound or the isomer.

14. A method for treating erythropenia, comprising administering a fused pyrazole compound of the formula 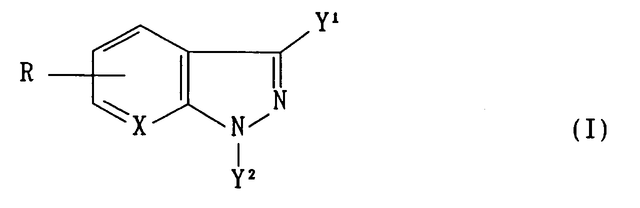 wherein
X is =N-;
R is a hydrogen, an alkyl, an alkoxy or a halogen;
Y¹ is a group of the formula 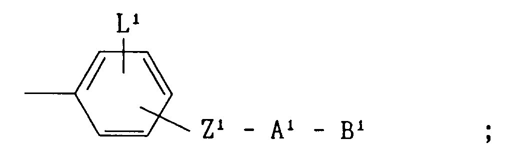 and
Y² is a group of the formula : -A²-B² or a hydrogen;
wherein Z¹ is -O-, A¹ and A² are the same or different and each is an alkylene, B¹ is a group of the formula 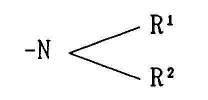 wherein R¹ and R² are the same or different and each is a hydrogen, an alkyl, a dialkylaminoalkyl or hydroxyalkyl, or R¹ and R² may form a heterocyclic ring together with the adjoining nitrogen atom, B² is a carboxyl, an alkoxycarbonyl, a phenylalkoxycarbonyl wherein phenyl nucleus may be substituted by 1 to 3 substituent(s) selected from the group consisting of halogen, nitro, amino, alkyl and alkoxy and L¹ is a hydrogen, a halogen, an alkyl or an alkoxy, an optical isomer thereof or a pharmaceutically acceptable salt of said compound or the isomer.

15. A method for treating etythropenia, comprising administering a compound selected from the group consisting of
3-[4-(2-dimethylamino-1-methylethoxy)phenyl]-1H-pyrazolo[3,4-b]pyridine-1-acetic acid,
(S)-3-[4-(2-dimethylamino-1-methylethoxy)phenyl]-1H-pyrazolo[3,4-b]pyridine-1-acetic acid,
ethyl 3-[4-(2-dimethylamino-1-methylethoxy)phenyl]-1H-pyrazolo[3,4-b]pyridine-1-acetate,
ethyl (S)-3-[4-(2-dimethylamino-1-methylethoxy)phenyl]-1H-pyrazolo[3,4-b]pyridine-1-acetate,
3-[4-(3-dimethylaminopropoxy)phenyl]-6-isopropyl-1H-pyrazolo[3,4-b]pyridine-1-acetic acid,
ethyl 3-[4-(3-dimethylaminopropoxy)phenyl]-6-isopropyl-1H-pyrazolo[3,4-b]pyridine-1-acetate,
3-[4-(2-dimethylamino-1-methylethoxy)phenyl]-6-isopropyl-1H-pyrazolo[3,4-b]pyridine-1-acetic acid,
(2)-3-[4-(2-dimethylamino-1-methylethoxy)phenyl]-6-isopropyl-1H-pyrazolo[3,4-b]pyridine-1-acetic acid,
ethyl 3-[4-(2-dimethylamino-1-methylethoxy)phenyl]-6-isopropyl-1H-pyrazolo[3,4-b]pyridine-1-acetate,
ethyl (S)-3-[4-(2-dimethylamino-1-methylethoxy)phenyl]-6-isopropyl-1H-pyrazolo[3,4-b]pyridine-1-acetate,
6-methyl-3-[4-(3-dimethylaminopropoxy)phenyl]-1H-pyrazolo[3,4-b]pyridine-1-acetic acid and
ethyl 6-methyl-3-[4-(3-dimethylaminopropoxy)phenyl]-1H-pyrazolo[3,4-b]pyridine-1-acetate, or a pharmaceutically acceptable salt thereof.

16. A use of a fused pyrazole compound of the formula 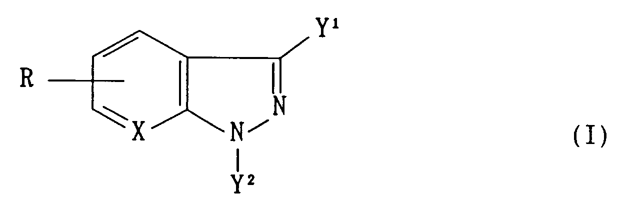 wherein
X is =N- or =CH-;
R is a hydrogen, an alkyl, an alkoxy or a halogen;
Y¹ is a group of the formula 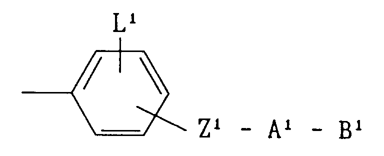 or
a phenyl which may be substituted by 1 to 3 substituent(s) selected from the group consisting of halogen, nitro, amino, alkyl and alkoxy; and
Y² is a group of the formula 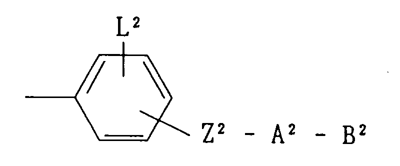 a group of the formula : -A²-B², a hydrogen, an alkyl, a phenylalkyl wherein phenyl nucleus may be substituted by 1 to 3 substituent(s) selected from the group consisting of halogen, nitro, amino, alkyl and alkoxy, a phenyl which may be substituted by 1 to 3 substituent(s) selected from the group consisting of halogen, nitro, amino, alkyl and alkoxy), an alkoxyalkyl, a hydroxyalkyl, an alkoxycarbonylalkyl, a carboxyalkyl or an acyloxyalkyl;
provided that either or both of Y¹ and Y² is(are) represented by the formula 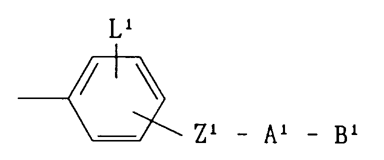 or the formula 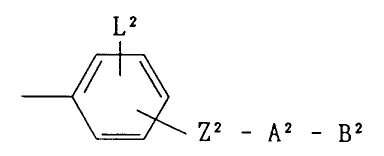 wherein Z¹ and Z² are the same or different and each is -O-, -S-or -NR³- wherein R³ is a hydrogen or an alkyl, A¹ and A² are the same or different and each is an alkylene, B¹ and B² are the same or different and each is a carboxyl, an alkoxycarbonyl, a phenylalkoxycarbonyl wherein phenyl nucleus may be substituted by 1 to 3 substituent(s) selected from the group consisting of halogen, nitro, amino, alkyl and alkoxy, an acyl, a hydroxyl, an acyloxy or a group of the formula 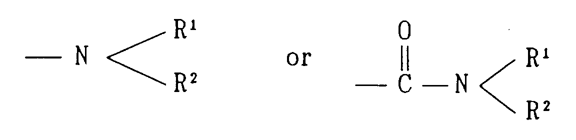 wherein R¹ and R² are the same or different and each is a hydrogen, an alkyl, a dialkylaminoalkyl or a hydroxyalkyl, or R¹ and R² may form a heterocyclic ring together with the adjoining nitrogen atom, and L¹ and L² are the same or different and each is a hydrogen, a halogen, an alkyl or an alkoxy, an optical isomer thereof or a pharmaceutically acceptable salt of said compound or the isomer for the production of an agent for treating erythropenia.

17. A use of a fused pyrazole compound of the formula 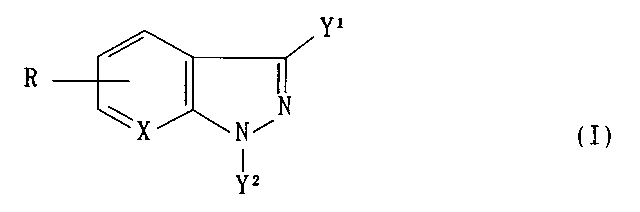 wherein
X is =N-;
R is a hydrogen, an alkyl, an alkoxy or a halogen;
Y¹ is a group of the formula 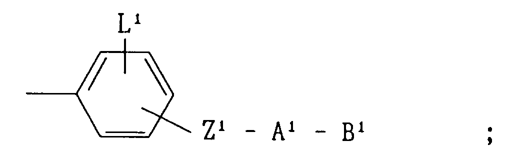 and
Y² is a group of the formula : -A²-B² or a hydrogen;
wherein Z¹ is -O-, A¹ and A² are the same or different and each is an alkylene, B¹ is a group of the formula 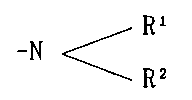 wherein R¹ and R² are the same or different and each is a hydrogen, an alkyl, a dialkylaminoalkyl or hydroxyalkyl, or R¹ and R² may form a heterocyclic ring together with the adjoining nitrogen atom, B² is a carboxyl, an alkoxycarbonyl or a phenylalkoxycarbonyl wherein phenyl nucleus may be substituted by 1 to 3 substituent(s) selected from the group consisting of halogen, nitro, amino, alkyl and alkoxy and L¹ is a hydrogen, a halogen, an alkyl or an alkoxy, an optical isomer thereof or a pharmaceutically acceptable salt of said compound or the isomer for the production of an agent for treating erythropenia.

18. A use of a compound selected from the group consisting of
3-[4-(2-dimethylamino-1-methylethoxy)phenyl]-1H-pyrazolo[3,4-b]pyridine-1-acetic acid,
(S)-3-[4-(2-dimethylamino-1-methylethoxy)phenyl]-1H-pyrazolo[3,4-b]pyridine-1-acetic acid,
ethyl 3-[4-(2-dimethylamino-1-methylethoxy)phenyl]-1H-pyrazolo[3,4-b]pyridine-1-acetate,
ethyl (S)-3-[4-(2-dimethylamino-1-methylethoxy)phenyl]-1H-pyrazolo[3,4-b]pyridine-1-acetate,
3-[4-(3-dimethylaminopropoxy)phenyl]-6-isopropyl-1H-pyrazolo[3,4-b]pyridine-1-acetic acid,
ethyl 3-[4-(3-dimethylaminopropoxy)phenyl]-6-isopropyl-1H-pyrazolo[3,4-b]pyridine-1-acetate,
3-[4-(2-dimethylamino-1-methylethoxy)phenyl]-6-isopropyl-1H-pyrazolo[3,4-b]pyridine-1-acetic acid,
(S)-3-[4-(2-dimethylamino-1-methylethoxy)phenyl]-6-isopropyl-1H-pyrazolo[3,4-b]pyridine-1-acetic acid,
ethyl 3-[4-(2-dimethylamino-1-methylethoxy)phenyl]-6-isopropyl-1H-pyrazolo[3,4-b]pyridine-1-acetate,
ethyl (S)-3-[4-(2-dimethylamino-1-methylethoxy)phenyl]-6-isopropyl-1H-pyrazolo[3,4-b]pyridine-1-acetate,
6-methyl-3-[4-(3-dimethylaminopropoxy)phenyl]-1H-pyrazolo[3,4-b]pyridine-1-acetic acid and
ethyl 6-methyl-3-[4-(3-dimethylaminopropoxy)phenyl]-1H-pyrazolo[3,4-b]pyridine-1-acetate, or a pharmaceutically acceptable salt thereof for the production of an agent for treating erythropenia.

19. (S)-3-[4-(2-Dimethylamino-1-methylethoxy)phenyl]-1H-pyrazolo[3,4-b]pyridine-1-acetic acid, an ethyl ester thereof or a pharmaceutically acceptable salt of said compound or the ethyl ester.
